# EUROPEAN PATENT APPLICATION

(11) **EP 4 385 992 A1**
(43) Date of publication of application: **19.06.2024**
(21) Application number: 22855509.0
(22) Date of filing: 11.08.2022
(51) Int. Cl.: C07D 471/04, A61K 31/5377, A61P 35/00

(54) **NAPHTHYRIDINE DERIVATIVE AS ATR INHIBITOR AND METHOD FOR PREPARING SAME**

(30) Priority: 11.08.2021 CN 202110919812; 12.10.2021 CN 202111188963; 01.08.2022 CN 202210918443
(71) Applicant: Wigen Biomedicine Technology (Shanghai) Co., Ltd., Shanghai 201203 (CN)
(72) Inventor: XIE, Yuli, Shanghai 201203 (CN); LIU, Wenzhong, Shanghai 201203 (CN); QIAN, Lihui, Shanghai 201203 (CN)
(74) Representative: Lipscombe, Martin John
(86) International application number: PCT/CN2022/111893
(87) International publication number: WO 2023/016529

(57) **Abstract**

Disclosed are a naphthyridine derivative as an ATR inhibitor, a method for preparing same, and use thereof. Specifically, the present invention relates to a naphthyridine compound of general formula (1), a method for preparing same, and use of the compound of general formula (1) and an isomer, a crystalline form, a pharmaceutically acceptable salt, a hydrate or a solvate thereof as an ATR inhibitor. The compound and the isomer, the crystalline form, the pharmaceutically acceptable salt, the hydrate or the solvate thereof of the present invention can be used for preparing a medicament for treating or preventing a related disease mediated by ATR protein kinase.

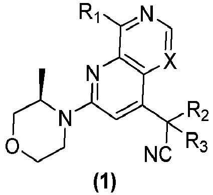

## Description

The present application claims priority to Chinese Patent Application No. 202110919812.6 filed on August 11, 2021, Chinese Patent Application No. 202111188963.5 filed on October 12, 2021, and Chinese Patent Application No. 202210918443.3 filed on August 1, 2022, which are incorporated herein by reference in their entirety.

### TECHNICAL FIELD

The present invention relates to the field of pharmaceutical chemistry, and in particular to a class of novel naphthyridine derivatives, a method for preparing same, and use thereof.

### BACKGROUND

Ataxia telangiectasia and Rad3-related (ATR) proteins, a member of the PIKK family, are a class of protein kinases involved in genome stability and DNA damage repair. Activation of ATR can be activated by stalled replication forks or by DNA single-strand breaks (SSBs). The activated ATR will recruit repair proteins or repair factors to repair the damaged parts and delay the mitosis process (especially in the G2/M phase of mitosis), which not only stabilizes the replication forks, but also ensures the genome stability.

Furthermore, the DNA damage repair system in most tumor cells is abnormal, and certain repair pathways (e.g., P53 or ATM mutations) are usually missing, making the tumor cells more dependent on ATR for survival. In normal cells, however, the inhibition of ATR kinase alone does not have a great effect due to the robust and intact repair pathway. Thus, the inhibition of ATR may have a more significant effect on the treatment of cancer without great toxic and side effects on normal cells.

DNA damage repair occurring during the S phase of the cell cycle is mainly accomplished by the ATR pathway, suggesting that ATR is very important to ensure cell proliferation. Analysis of clinical tumor samples indicates that elevated ATR expression levels are observed in a variety of tumor tissues, such as gastric cancer, liver cancer, colorectal cancer, ovarian cancer, pancreatic cancer, and the like. Moreover, in patients with ovarian cancer and pancreatic cancer, high level of ATR is usually associated with relatively low survival rates.

Furthermore, the inhibition of ATR can be combined with radiotherapy or chemotherapeutic drugs to enhance the effect synergistically. A wide variety of chemotherapeutic drugs include antimetabolites (e.g., gemcitabine), DNA cross-linking agents (e.g., cisplatin and carboplatin), alkylating agents (e.g., temozolomide), topoisomerase inhibitors (e.g., irinotecan), and the like. Tumor cells, when affected by chemotherapy or radiotherapy, will activate the ATR signaling pathway to a greater extent to repair the damaged DNA. Therefore, when using radiotherapy or chemotherapeutic drugs to treat cancer, inhibiting ATR at the same time can greatly enhance the therapeutic effect on cancer. Based on the above studies, ATR can be seen as an important target for effective tumor treatment.

Currently, compounds such as AZD6738 from AstraZeneca, VE822 from Merck, and BAY1895344 from Bayer have been subjected to phase I/II clinical studies.

### SUMMARY

The present invention provides a compound of general formula (1) or an isomer, a crystalline form, a pharmaceutically acceptable salt, a hydrate or a solvate thereof: 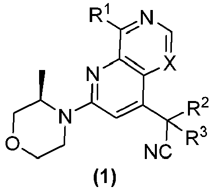 wherein in general formula (1):
X is CH or N;
R¹ is 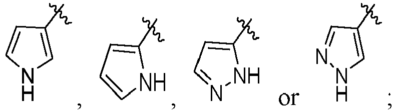
R² and R³ are each independently -H, -D, halogen, (C1-C6) alkyl, (C2-C6) alkenyl, (C2-C6) alkynyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl, wherein the (C1-C6) alkyl, (C2-C6) alkenyl, (C2-C6) alkynyl, (C1-C6) alkoxy, (C3-C10) cycloalkyl, (C3-C10) cycloalkenyl, (3- to 10-membered) heterocycloalkyl, (3-to 10-membered) heterocycloalkenyl, (C6-C10) aryl or (5- to 10-membered) heteroaryl can each independently be optionally substituted with one or more of the following groups: -H, -D, halogen, -OH, -R⁶, -NR⁴R⁵, -C(O)OR⁴, -C(O)NR⁴R⁵, -S(O)ₚR⁴, -S(O)₂NR⁴R⁵, -P(O)(OR⁴)₂, - P(O)(R⁴)₂, (C1-C6) alkyl, (C2-C6) alkenyl, (C2-C6) alkynyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
or R² and R³, together with the carbon atom to which they are attached, form a (C3-C15) cycloalkyl or (3- to 15-membered) heterocycloalkyl, wherein the (C3-C15) cycloalkyl or (3- to 15-membered) heterocycloalkyl can each independently be optionally substituted with one or more R⁶;
R⁴ and R⁵ are each independently -H, -D, (C1-C3) alkyl, (C2-C4) alkenyl, (C2-C4) alkynyl, (C1-C3) alkoxy, (C1-C3) haloalkyl, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
or R⁴ and R⁵, with the attached N atom, form a (3- to 10-membered) heterocycloalkyl, wherein the (3- to 10-membered) heterocycloalkyl can be optionally substituted with one or more of the following groups: -H, -D, halogen, -OH, -NR⁷R⁸, -C(O)OR⁷, -C(O)NR⁷R⁸, -S(O)ₚR⁷, - S(O)₂NR⁷R⁸, -P(O)(OR⁷)₂, -P(O)(R⁷)₂, (C1-C6) alkyl, (C2-C6) alkenyl, (C2-C6) alkynyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
each R⁶ is independently -H, -D, halogen, -OH, -NR⁷R⁸, -C(O)R⁷, -C(O)OR⁷, -C(O)NR⁷R⁸, - (CH₂)ₙ-S(O)ₚR⁷, -(CH₂)ₙ-S(O)₂NR⁷R⁸, -P(O)(OR⁷)₂, -P(O)(R⁷)₂, (C1-C6) alkyl, (C2-C6) alkenyl, (C2-C6) alkynyl, (C1-C6) haloalkyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl, wherein the (C1-C6) alkyl, (C2-C6) alkenyl, (C2-C6) alkynyl, (C1-C6) haloalkyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl can each independently be optionally substituted with one or more of the following groups: -H, -D, halogen, -OH, -NR⁷R⁸, -C(O)OR⁷, -C(O)NR⁷R⁸, -(CH₂)ₙ-S(O)ₚR⁷, -(CH₂)ₙ-S(O)₂NR⁷R⁸, -P(O)(OR⁷)₂, -P(O)(R⁷)₂, (C1-C6) alkyl, (C2-C6) alkenyl, (C2-C6) alkynyl, (C1-C6) haloalkyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
R⁷ and R⁸ are each independently -H, -D, (C1-C3) alkyl, (C2-C4) alkenyl, (C2-C4) alkynyl, (C1-C3) alkoxy, (C1-C3) haloalkyl, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
p is 0, 1 or 2;
n is 0, 1, 2 or 3.

In another embodiment of the present invention, in general formula (1), X is CH.

In another embodiment of the present invention, in general formula (1), R¹ is

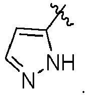

In another embodiment of the present invention, in general formula (1), R² and R³ are each independently -H, -D, -F, -Cl, -Br, -I, (C1-C3) alkyl, (C2-C4) alkenyl, (C2-C4) alkynyl, (C1-C3) alkoxy, (C3-C6) cycloalkyl, (C3-C6) cycloalkenyl, (3- to 8-membered) heterocycloalkyl, (3- to 8-membered) heterocycloalkenyl, (C6-C10) aryl or (5- to 10-membered) heteroaryl, wherein the (C1-C3) alkyl, (C2-C4) alkenyl, (C2-C4) alkynyl, (C1-C3) alkoxy, (C3-C6) cycloalkyl, (C3-C6) cycloalkenyl, (3- to 8-membered) heterocycloalkyl, (3- to 8-membered) heterocycloalkenyl, (C6-C10) aryl or (5- to 10-membered) heteroaryl can each independently be optionally substituted with one or more of the following groups: -H, -D, -F, -Cl, -Br, -I, -OH, -R⁶, -NR⁴R⁵, -C(O)OR⁴, -C(O)NR⁴R⁵, -S(O)ₚR⁴, -S(O)₂NR⁴R⁵, -P(O)(OR⁴)₂, -P(O)(R⁴)₂, (C1-C3) alkyl, (C2-C4) alkenyl, (C2-C4) alkynyl, (C1-C3) alkoxy, (C3-C6) cycloalkyl, (C3-C6) cycloalkenyl, (3-to 8-membered) heterocycloalkyl, (3- to 8-membered) heterocycloalkenyl, (C6-C10) aryl or (5-to 10-membered) heteroaryl; or R² and R³, together with the carbon atom to which they are attached, form a (C3-C15) cycloalkyl or (3- to 15-membered) heterocycloalkyl, wherein the (C3-C15) cycloalkyl or (3- to 15-membered) heterocycloalkyl can each independently be optionally substituted with one or more R⁶.

In another embodiment of the present invention, in general formula (1), R² and R³ are each independently -H, -D, -F, -Cl, -Br, -I, -CH₃, 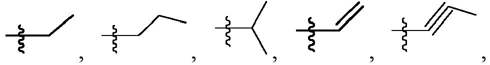 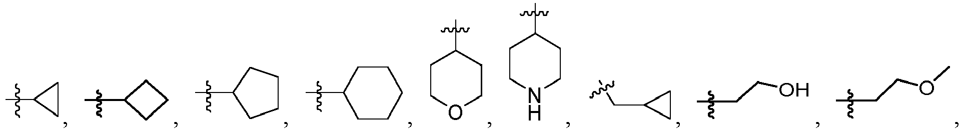 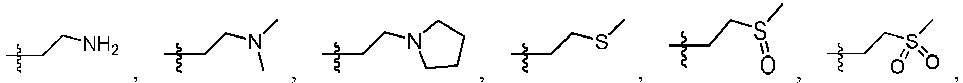 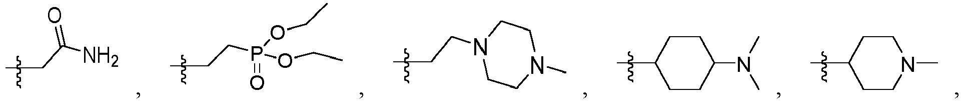 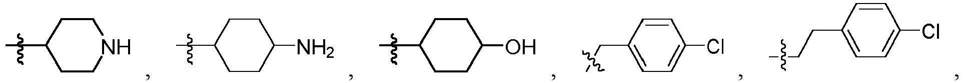 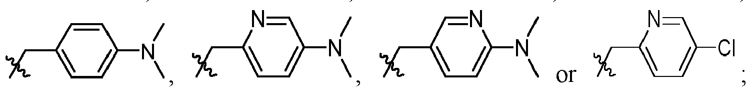 preferably, R² is -H or -CH₃, and R³ is -H, -F, -Cl, -CH₃, 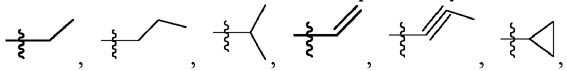 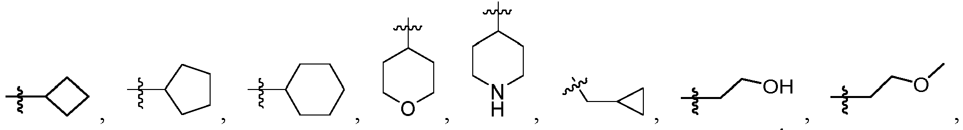 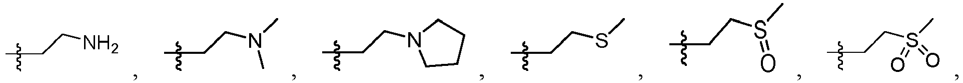 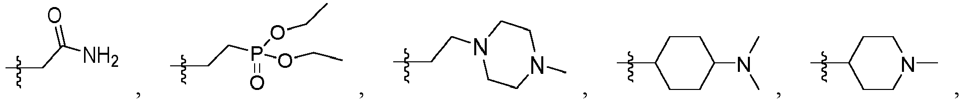 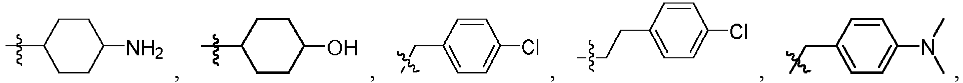 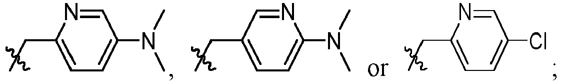 more preferably, R² is -H or -CH₃, and R³ is -H, -CH₃,

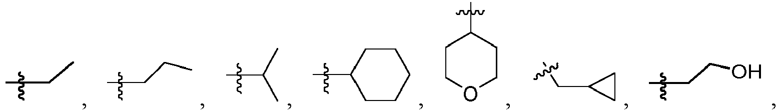

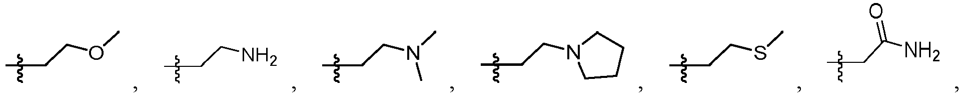

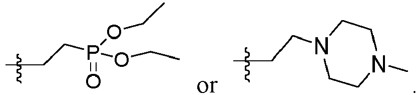

In another embodiment of the present invention, in general formula (1), the (C3-C15) cycloalkyl or (3- to 15-membered) heterocycloalkyl formed by R² and R³ together with the carbon atom to which they are attached is: 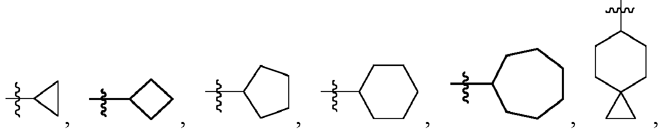 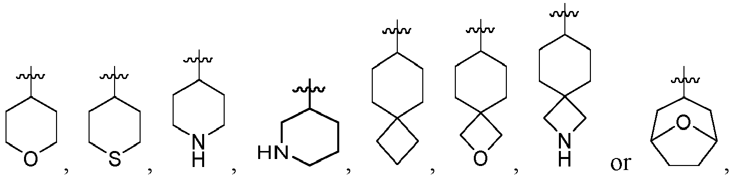 preferably, 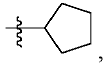 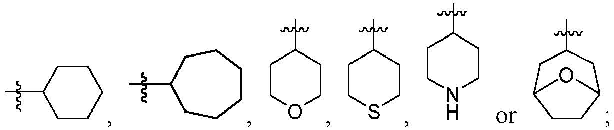 the (C3-C15) cycloalkyl or (3- to 15-membered) heterocycloalkyl can be optionally substituted with one or more of the following groups: -H, -F, -CH₃, -CH₂CH₃, -OH, 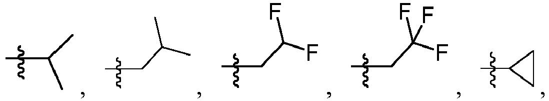 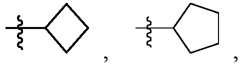 -NH₂, -NH(CH₃), -N(CH₃)₂, -N(CH₂CH₃)₂, -OCH₃, -OCH₂CH₃, 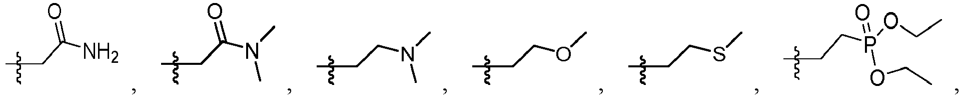 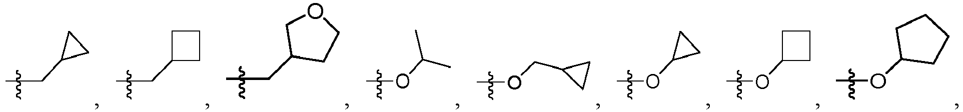 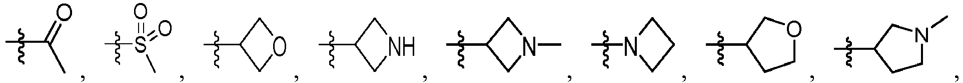 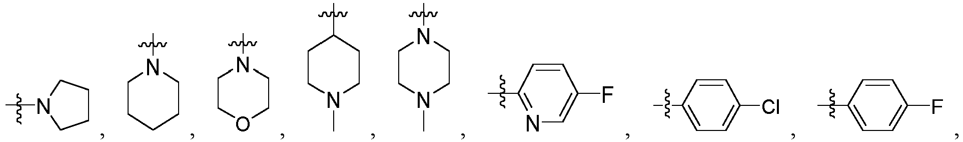 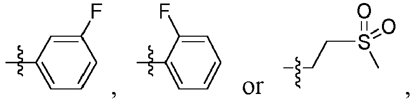 preferably -H, -F, -CH₃, -CH₂CH₃, -OH, 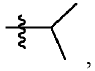 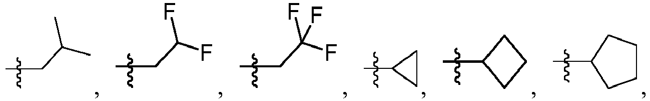 -NH₂, -NH(CH₃), -N(CH₃)₂, - N(CH₂CH₃)₂, -OCH₃, -OCH₂CH₃,

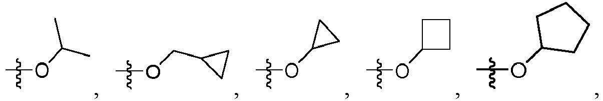

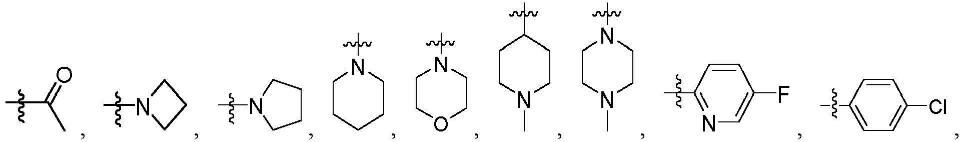

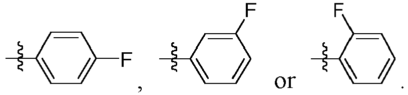

In another embodiment of the present invention, in general formula (1), R⁴ and R⁵ are each independently -H, -D, -CH₃,

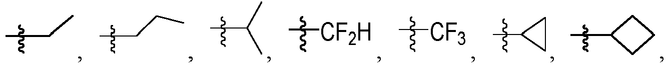

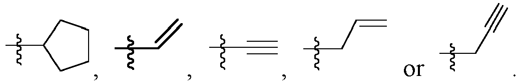

In another embodiment of the present invention, in general formula (1), R⁴ and R⁵ are each independently -H or -CH₃.

In another embodiment of the present invention, in general formula (1), R⁶ is -H, -F, -CH₃, - CH₂CH₃, -OH, 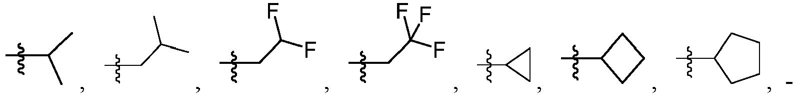 NH₂, -NH(CH₃), -N(CH₃)₂, -N(CH₂CH₃)₂, -OCH₃, -OCH₂CH₃, 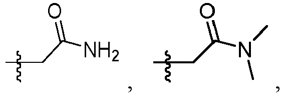 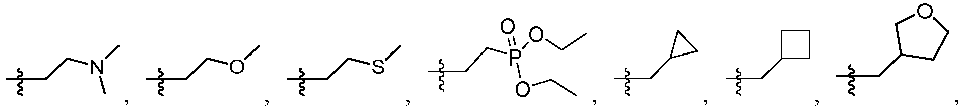 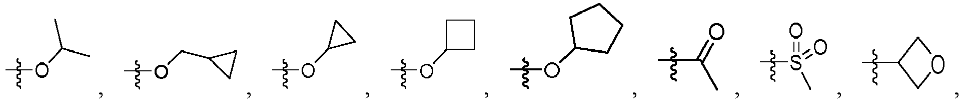 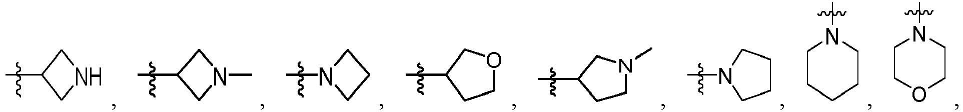 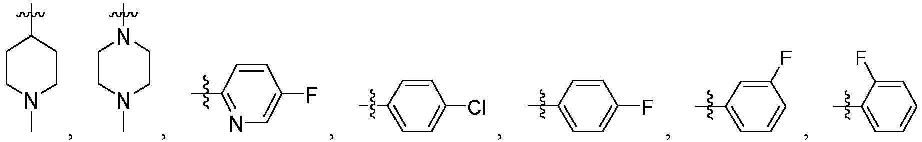 or 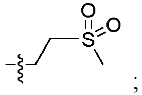 preferably -H, -F, -CH₃, -CH₂CH₃, -OH, 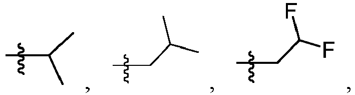 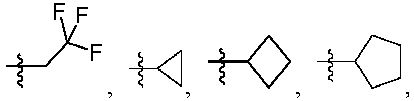 -NH₂, -NH(CH₃), -N(CH₃)₂, -N(CH₂CH₃)₂, -OCH₃, - OCH₂CH₃,

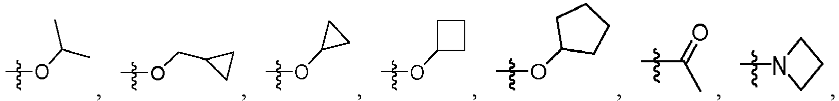

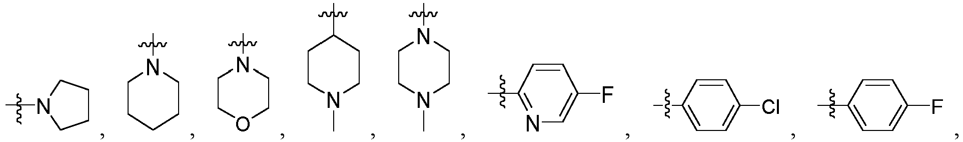

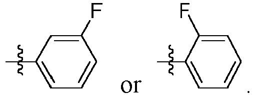

In another embodiment of the present invention, in general formula (1), R⁷ and R⁸ are each independently -H or -CH₃.

In another specific embodiment of the present invention, the compound of general formula (1) has one of the following structures: 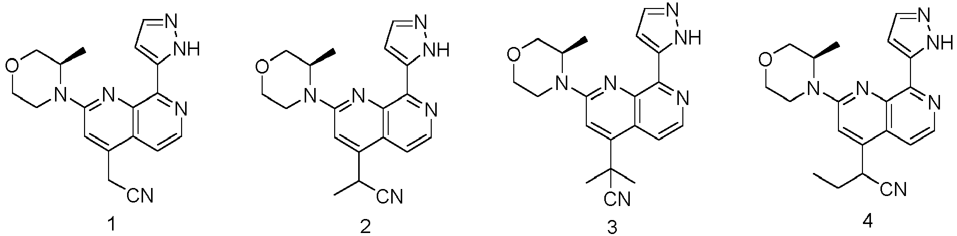 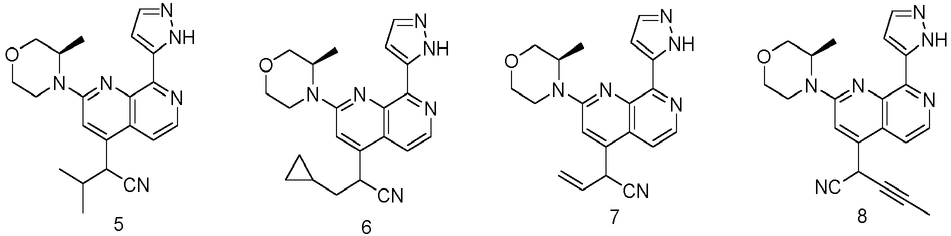 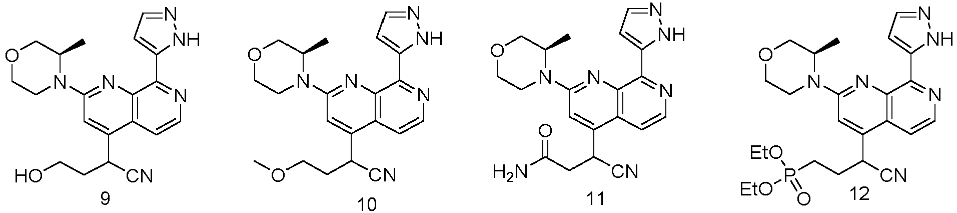 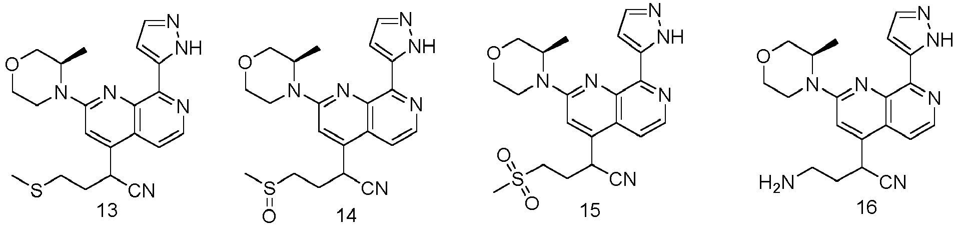 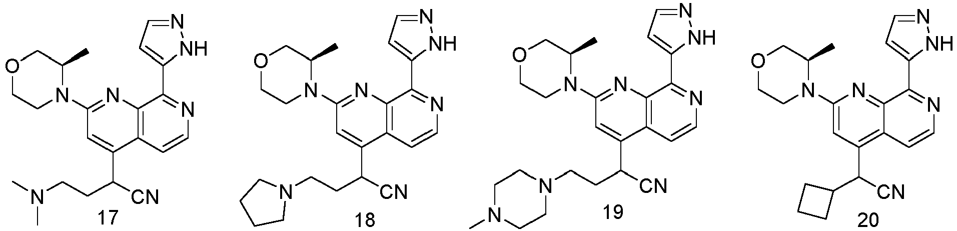 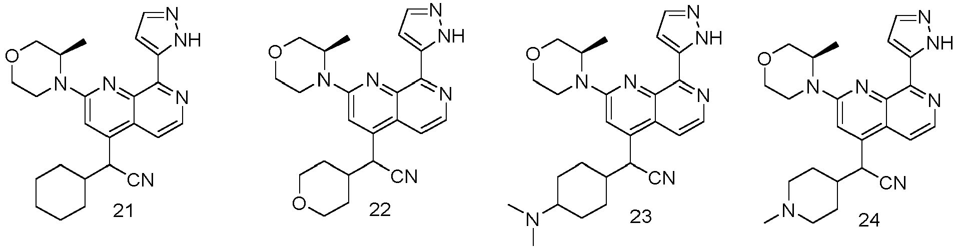 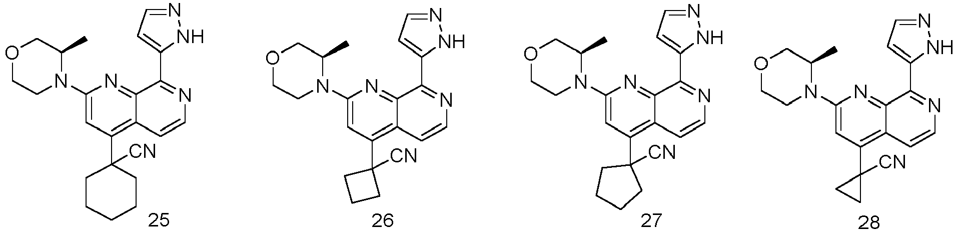 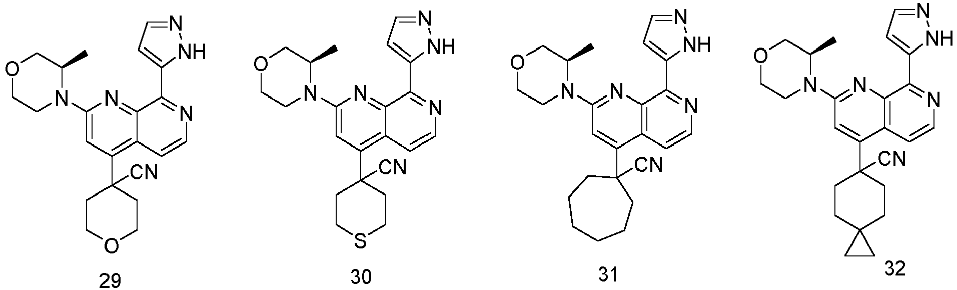 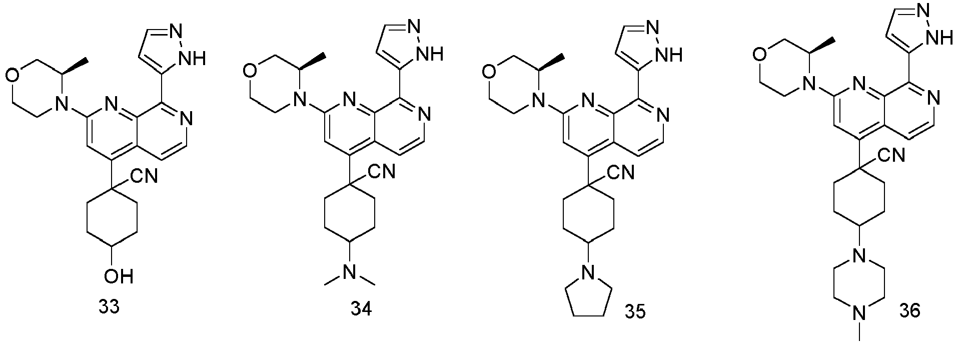 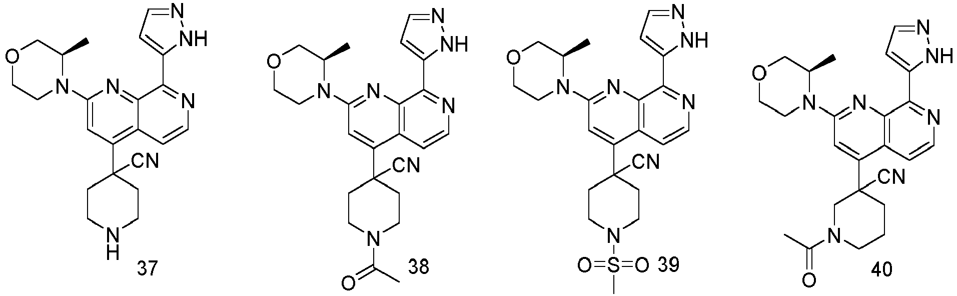 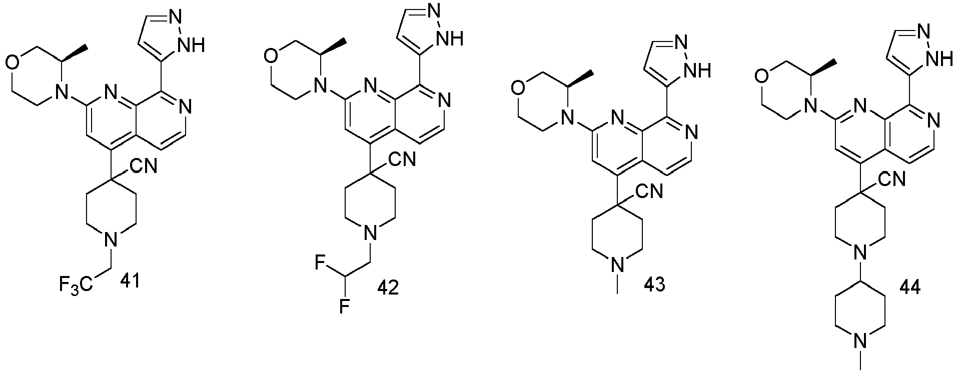 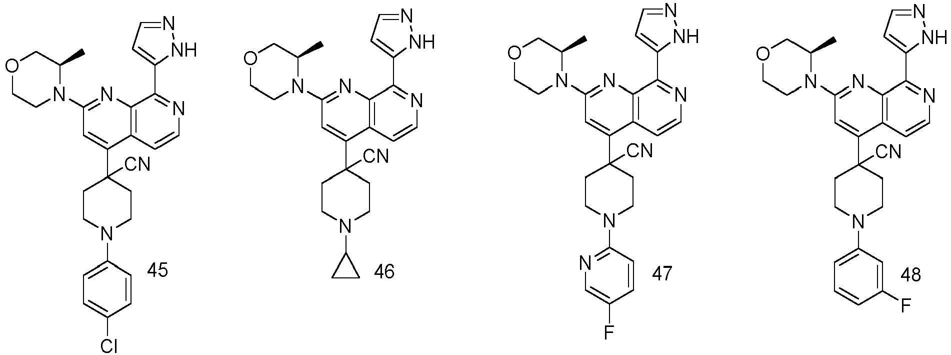 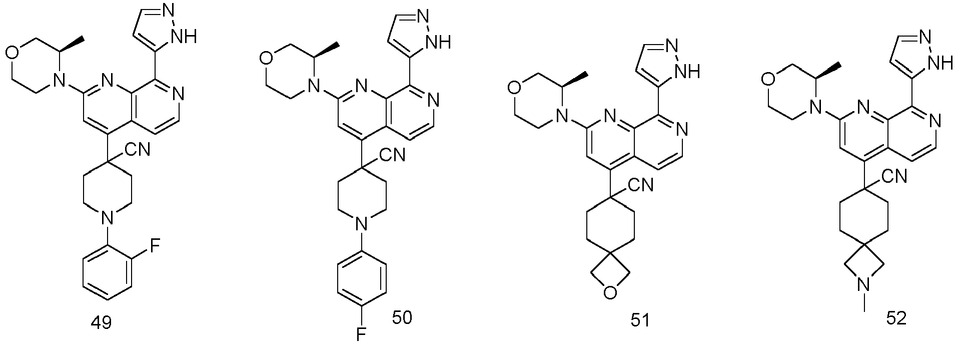 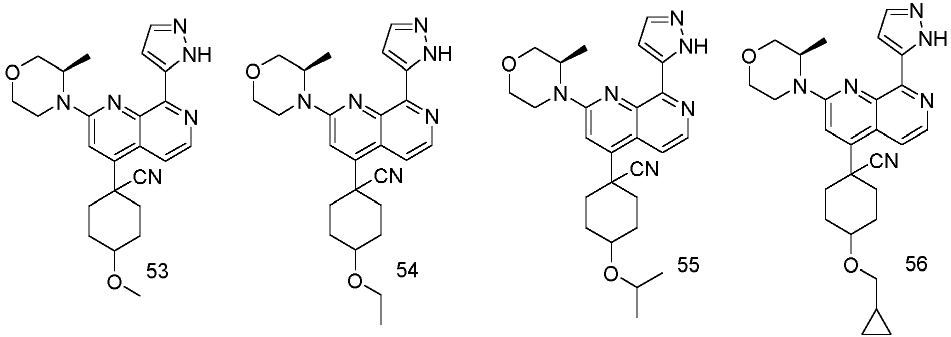 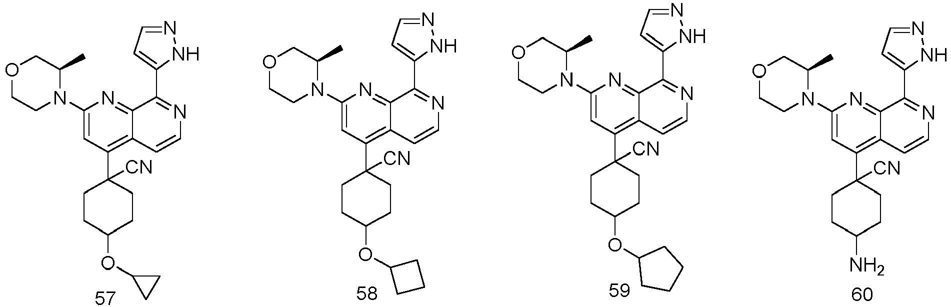 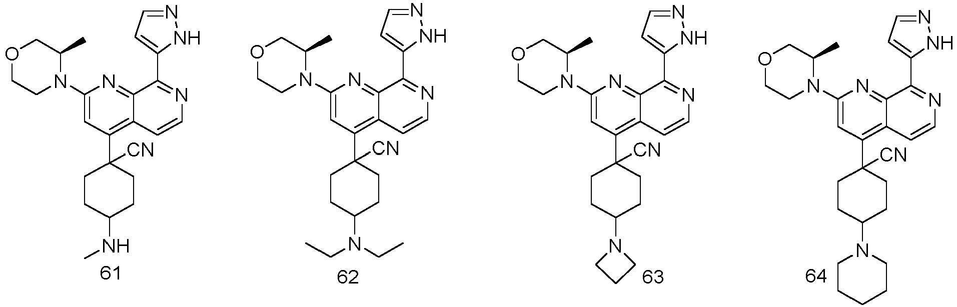 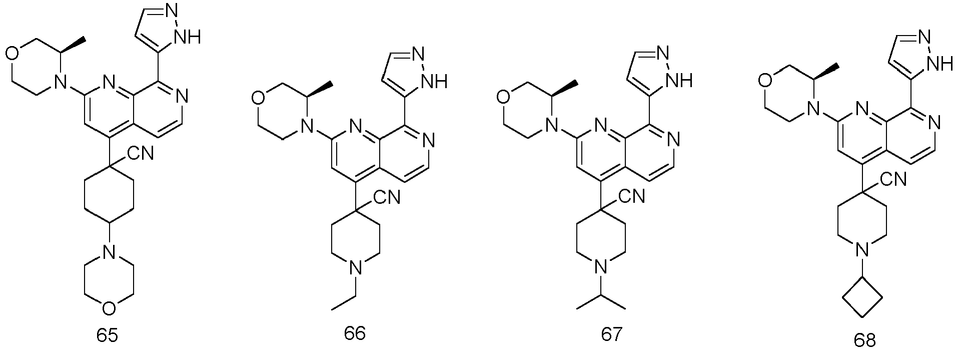 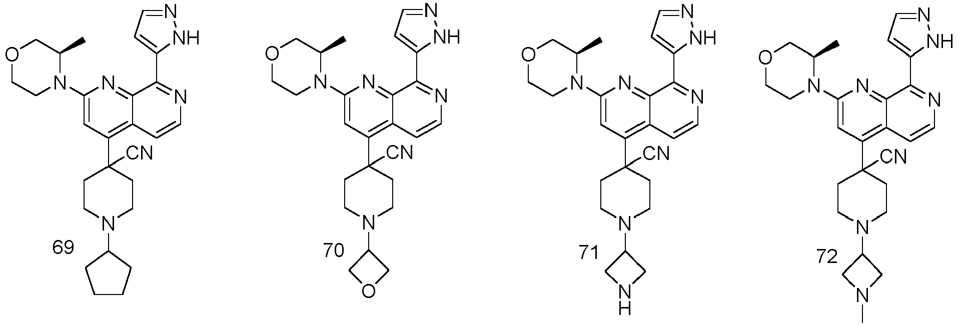 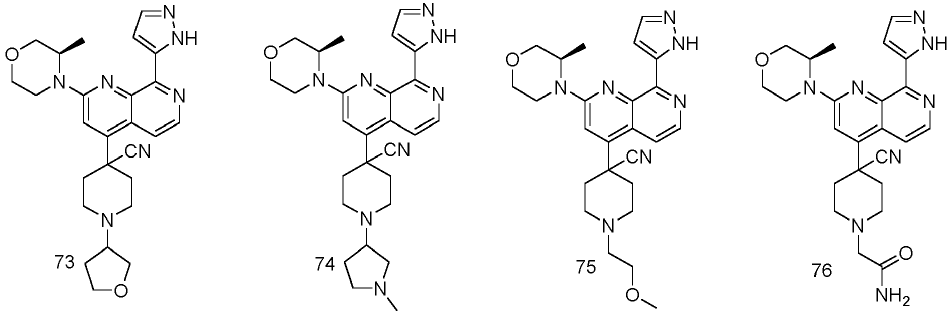 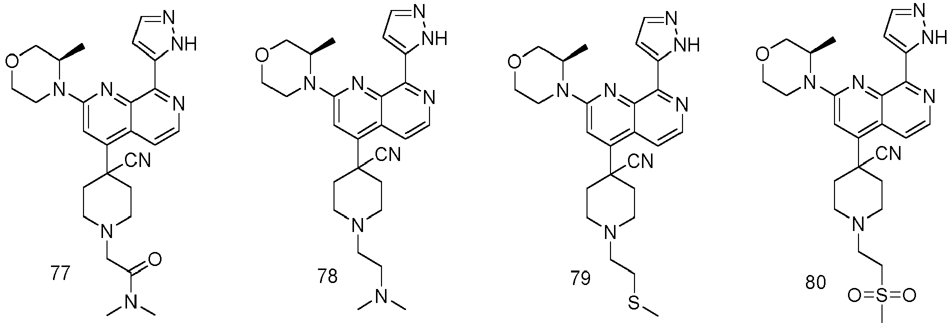 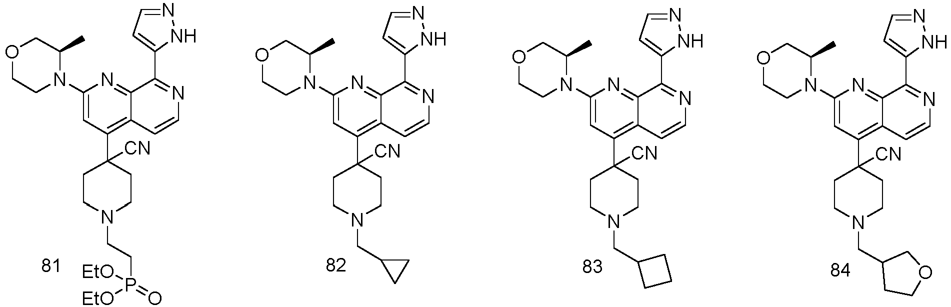 or

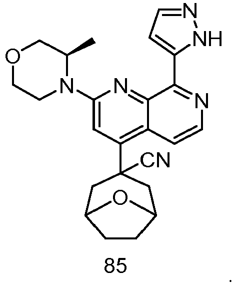

Another object of the present invention is to provide a pharmaceutical composition comprising a pharmaceutically acceptable carrier, diluent and/or excipient, and the compound of general formula (1) or the isomer, the crystalline form, the pharmaceutically acceptable salt, the hydrate or the solvate thereof of the present invention as an active ingredient.

The present invention is still further intended to provide use of the compound of general formula (1) or the isomer, the crystalline form, the pharmaceutically acceptable salt, the hydrate or the solvate thereof of the present invention, or the pharmaceutical composition described above in the preparation of a medicament for treating, regulating or preventing a disease related to ATR protein kinase.

The present invention is even further intended to provide a method for treating, regulating or preventing a related disease mediated by ATR protein kinase, the method including administering to a subject a therapeutically effective amount of the compound of general formula (1) or the isomer, the crystalline form, the pharmaceutically acceptable salt, the hydrate or the solvate of the present invention, or the pharmaceutical composition described above.

It should be understood that both the above general description and the following detailed description of the present invention are exemplary and explanatory, and are intended to provide further explanation of the present invention claimed.

### Synthesis of Compounds

Methods for preparing the compounds of general formula (1) of the present invention are specifically described below, but these specific methods do not limit the present invention in any way.

The compounds of general formula (1) described above can be synthesized using standard synthetic techniques or well-known techniques in combination with the methods described herein. In addition, the solvents, temperatures and other reaction conditions mentioned herein may vary. Starting materials for the synthesis of the compounds can be obtained synthetically or commercially. The compounds described herein and other related compounds with different substituents can be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing the compounds can be changed by using appropriate reagents and conditions for introducing different groups into the molecular formulas provided herein.

In one aspect, the compounds described herein are prepared according to methods well known in the art. However, the conditions of the methods, such as reactants, solvents, bases, the amount of the compounds used, reaction temperature and time required for the reaction are not limited to the following explanation. The compounds of the present invention can also be conveniently prepared by optionally combining various synthetic methods described herein or known in the art, and such combinations can be easily determined by those skilled in the art to which the present invention pertains. In one aspect, the present invention further provides a method for preparing the compound of general formula (1), wherein the compound of general formula (1) can be prepared using method A, method B or method C below:

### Method A

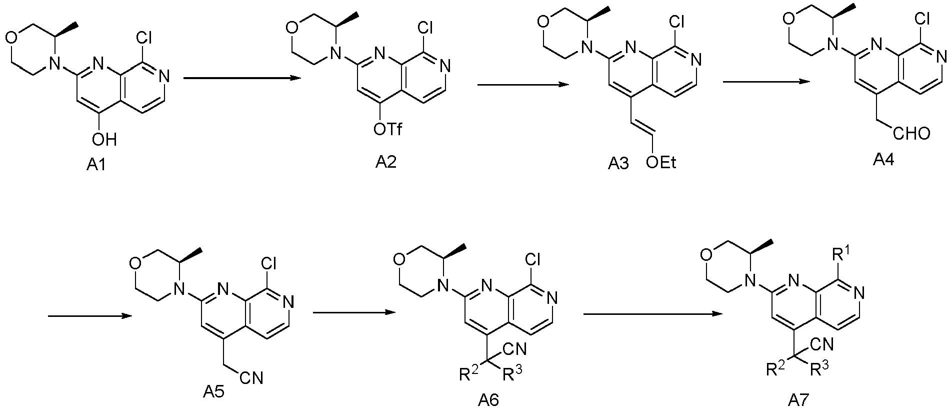

The compound of general formula (1) can be prepared according to method A, wherein R¹, R² and ³ are as defined above.

Method A includes the following steps: firstly, reacting a compound A1 under an alkaline condition to generate a compound A2; further subjecting the compound A2 to a metal-catalyzed coupling reaction to generate a compound A3; subj ecting the compound A3 to deprotection under an acidic condition to generate A4; reacting A4 with a cyanation reagent to generate A5; reacting A5 with a strong alkali and a halogenating reagent to generate A6; and subjecting A6 to a coupling reaction to generate a target compound A7.

### Method B

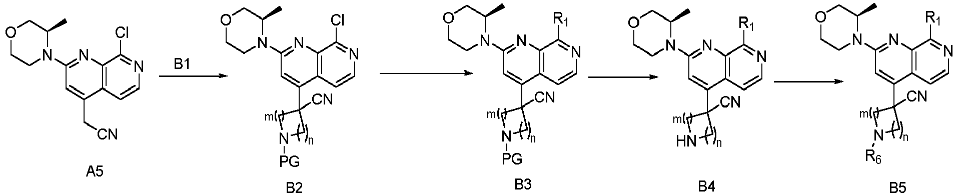

The compound of general formula (1) can also be prepared according to method B, wherein n and m represent 0, 1, 2 or 3; PG represents an N-protecting group; R¹ and R⁶ are as defined above.

Method B includes the following steps: firstly, reacting a compound A5 with a fragment B 1 under the action of a strong alkali to generate B2; subjecting B2 to a coupling reaction to generate B3; subjecting B3 to deprotection under an appropriate condition to form B4; and further reacting B4 to give a target compound B6.

### Method C

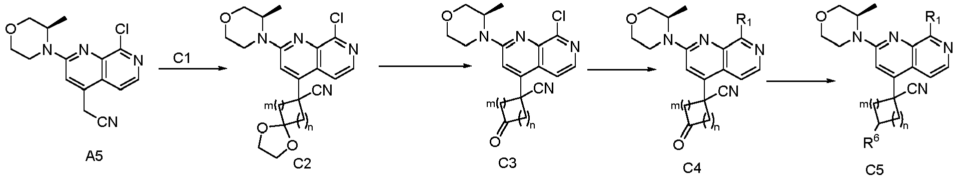

The compound of general formula (1) can also be prepared according to method C, wherein n and m represent 0, 1, 2 or 3; R¹ and R⁶ are as defined above.

Method C includes the following steps: firstly, reacting a compound A5 with a fragment C1 under the action of a strong alkali to generate C2; subjecting C2 to deprotection by an acid to generate C3; subjecting C3 to a coupling reaction to generate C4; and reacting C4 under an appropriate condition to give a target compound C5.

### Further Forms of the Compounds

"Pharmaceutically acceptable" herein refers to a substance, such as a carrier or diluent, which will not lead to loss of biological activity or properties of a compound and is relatively non-toxic. For example, when an individual is given a substance, the substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of general formula (1) to a reaction with acids, e.g., inorganic acids such as hydrochloric acid, hydrobromic acid, hydrofluoric acid, sulfuric acid, phosphoric acid, nitric acid, carbonic acid and the like; organic acids such as formic acid, acetic acid, propionic acid, oxalic acid, trifluoroacetic acid, malonic acid, succinic acid, fumaric acid, maleic acid, lactic acid, malic acid, tartaric acid, citric acid, picric acid, methanesulfonic acid, benzenesulfonic acid, *p*-toluenesulfonic acid and the like; and acidic amino acids such as aspartic acid, glutamic acid and the like.

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to the methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized and nanoparticle forms. In addition, the compound of general formula (1) includes crystalline forms, and may also be polymorphs. Polymorphs include different lattice arrangements of the same elements of a compound. The polymorphs generally have different X-ray diffraction spectra, infrared spectra, melting points, density, hardness, crystalline forms, optical and electrical properties, stability and solubility.

Different factors such as a recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer and a cis-trans isomer. Each chiral center or axial chirality will independently produce two optical isomers, and all possible optical isomers, diastereomeric mixtures, and pure or partially pure compounds are included within the scope of the present invention. The present invention is meant to include all such isomeric forms of these compounds.

The compound of the present invention may contain unnatural proportions of atomic isotopes at one or more of the atoms that constitute the compound. For example, the compound may be labeled with radioactive isotopes, such as tritium (³H), iodine-125 (¹²⁵I) and C-14 (¹⁴C). For another example, deuterium can be used to substitute a hydrogen atom to form a deuterated compound. The bond formed by deuterium and carbon is stronger than that formed by ordinary hydrogen and carbon. Compared with an undeuterated medicament, the deuterated medicament generally has the advantages of reduced toxic and side effects, increased pharmaceutical stability, enhanced efficacy, prolonged pharmaceutical *in vivo* half-life and the like. All isotopic variations of the compound of the present invention, whether radioactive or not, are contained within the scope of the present invention.

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA technology and pharmacology are used. As used herein, "or" or "and" refers to "and/or" unless otherwise stated.

Unless otherwise specified, "alkyl" refers to a saturated aliphatic hydrocarbon group, including linear and branched groups containing 1 to 6 carbon atoms. Lower alkyl groups containing 1 to 4 carbon atoms, such as methyl, ethyl, propyl, 2-propyl, *n*-butyl, isobutyl, or *tert*-butyl, are preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, CF₃(CH₃)CH, ⁱPr, ⁿPr, ⁱBu, ⁿBu or ^{t}Bu.

Unless otherwise specified, "alkylene" refers to a divalent alkyl as defined above. Examples of alkylene include, but are not limited to, methylene and ethylene.

Unless otherwise specified, "alkenyl" refers to an unsaturated aliphatic hydrocarbon group containing carbon-carbon double bonds, including linear or branched groups containing 1 to 14 carbon atoms. Lower alkenyl groups containing 1 to 4 carbon atoms, such as vinyl, 1-propenyl, 1-butenyl, or 2-methylpropenyl, are preferred.

Unless otherwise specified, "alkynyl" refers to an unsaturated aliphatic hydrocarbon group containing carbon-carbon triple bonds, including linear and branched groups containing 1 to 14 carbon atoms. Lower alkynyl groups containing 1 to 4 carbon atoms, such as ethynyl, 1-propynyl, or 1-butynyl, are preferred.

Unless otherwise specified, "cycloalkyl" refers to a non-aromatic hydrocarbon ring system (monocyclic, bicyclic or polycyclic), and partially unsaturated cycloalkyl may be referred to as "cycloalkenyl" if the carbocyclic ring contains at least one double bond, or "cycloalkynyl" if the carbocyclic ring contains at least one triple bond. Cycloalkyl may include monocyclic or polycyclic groups and spiro rings (e.g., having 2, 3 or 4 fused rings). In some embodiments, cycloalkyl is monocyclic. In some embodiments, cycloalkyl is monocyclic or bicyclic. The ring carbon atoms of cycloalkyl may optionally be oxidized to form an oxo or sulfido group. Cycloalkyl further includes cycloalkylene. In some embodiments, cycloalkyl contains 0, 1 or 2 double bonds. In some embodiments, cycloalkyl contains 1 or 2 double bonds (partially unsaturated cycloalkyl). In some embodiments, cycloalkyl may be fused to aryl, heteroaryl, cycloalkyl and heterocycloalkyl. In some embodiments, cycloalkyl may be fused to aryl, cycloalkyl and heterocycloalkyl. In some embodiments, cycloalkyl may be fused to aryl and heterocycloalkyl. In some embodiments, cycloalkyl may be fused to aryl and cycloalkyl. Examples of cycloalkyl include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcarnyl, bicyclo[1.1.1]pentyl, bicyclo[2.1.1]hexyl and the like.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, *sec*-butoxy and *tert*-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO or ^{t-}BuO.

Unless otherwise specified, "aryl" refers to an aromatic hydrocarbon group, which is monocyclic or polycyclic; for example, a monocyclic aryl ring may be fused to one or more carbocyclic aromatic groups. Examples of aryl include, but are not limited to, phenyl, naphthyl, and phenanthryl.

Unless otherwise specified, "aryloxy" refers to an aryl group that bonds to the rest of the molecule through an ether oxygen atom. Examples of aryloxy include, but are not limited to, phenoxy and naphthoxy.

Unless otherwise specified, "arylene" refers to a divalent aryl as defined above. Examples of arylene include, but are not limited to, phenylene, naphthylene, and phenanthrylene.

Unless otherwise specified, "heteroaryl" refers to an aromatic group containing one or more heteroatoms (O, S, or N), and the "heteroaryl" is monocyclic or polycyclic. For example, a monocyclic heteroaryl ring is fused to one or more carbocyclic aromatic groups or other monocyclic heterocycloalkyl groups. Examples of heteroaryl include, but are not limited to, pyridinyl, pyridazinyl, imidazolyl, pyrimidinyl, pyrazolyl, triazolyl, pyrazinyl, quinolinyl, isoquinolinyl, furanyl, thienyl, isoxazolyl, thiazolyl, oxazolyl, isothiazolyl, pyrrolyl, indolyl, benzimidazolyl, benzofuranyl, benzothiazolyl, benzothienyl, benzoxazolyl, benzopyridinyl, pyrrolopyrimidinyl, 1*H*-pyrrolo[3,2-b]pyridinyl, 1*H*-pyrrolo[2,3-c]pyridinyl, 1*H*-pyrrolo[3,2-c]pyridinyl, 1*H*-pyrrolo[2,3-b]pyridinyl, 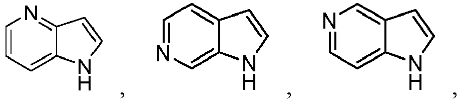 and

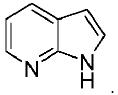

Unless otherwise specified, "heterocycloalkyl" refers to a non-aromatic ring or ring system, which may optionally contain one or more alkenylene as part of the ring structure, having at least one heteroatom ring member independently selected from boron, phosphorus, nitrogen, sulfur, oxygen, and phosphorus. Partially unsaturated heterocycloalkyl may be referred to as "heterocycloalkenyl" if heterocycloalkyl contains at least one double bond, or "heterocycloalkynyl" if the heterocycloalkyl contains at least one triple bond. Heterocycloalkyl may include monocyclic, bicyclic, spiro ring, or polycyclic systems (e.g., having two fused or bridged rings). In some embodiments, heterocycloalkyl is a monocyclic group having 1, 2, or 3 heteroatoms independently selected from nitrogen, sulfur, and oxygen. The ring carbon atoms and heteroatoms of heterocycloalkyl may optionally be oxidized to form oxo or sulfido groups or other oxidized bonds (e.g., C(O), S(O), C(S) or S(O)2, N-oxides, etc.), or the nitrogen atoms may be quaternized. Heterocycloalkyl may be attached via a ring carbon atom or a ring heteroatom. In some embodiments, heterocycloalkyl contains 0 to 3 double bonds. In some embodiments, heterocycloalkyl contains 0 to 2 double bonds. The definition of heterocycloalkyl further includes moieties having one or more aromatic rings fused to (i.e., sharing a bond with) the heterocycloalkyl ring, for example, benzo-derivatives of piperidine, morpholine, azepin, thienyl, or the like. Heterocycloalkyl containing a fused aromatic ring may be attached via any ring atom, including ring atoms of the fused aromatic ring. Examples of heterocycloalkyl include, but are not limited to, azetidinyl, azepinyl, dihydrobenzofuranyl, dihydrofuranyl, dihydropyranyl, *N*-morpholinyl, 3-oxa-9-azaspiro[5.5]undecyl, 1-oxa-8-azaspiro[4.5]decyl, piperidinyl, piperazinyl, oxopiperazinyl, pyranyl, pyrrolidinyl, quininyl, tetrahydrofuranyl, tetrahydropyranyl, 1,2,3,4-tetrahydroquinolinyl, tropanyl, 4,5,6,7-tetrahydrothiazolo[5,4-c]pyridinyl, 4,5,6,7-tetrahydro-1*H*-imidazo[4,5-c]pyridine, *N*-methylpiperidinyl, tetrahydroimidazolyl, pyrazolidinyl, butyrolactam, valerolactam, imidazolidinonyl, hydantoinyl, dioxolanyl, phthalimidyl, pyrimidine-2,4(1*H*,3*H*)-dione, 1,4-dioxanyl, morpholinyl, thiomorpholinyl, thiomorpholinyl-S-oxide, thiomorpholinyl-*S*,*S*-oxide, piperazinyl, pyranyl, pyridonyl, 3-pyrrolinyl, thiopyranyl, pyronyl, tetrahydrothienyl, 2-azaspiro[3.3]heptanyl, indolinyl,

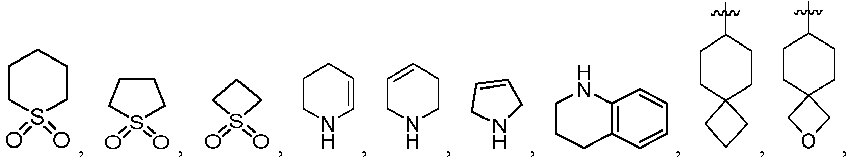

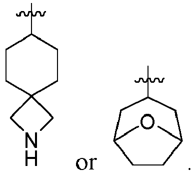

Unless otherwise specified, "halogen" (or halo) refers to fluorine, chlorine, bromine or iodine. The term "halo" (or "halogenated") before a group name indicates that the group is partially or fully halogenated, that is, substituted in any combination with F, Cl, Br or I, preferably with F or Cl.

"Optional" or "optionally" means that the subsequently described event or circumstance may, but does not necessarily, occur, and the description includes instances where the event or circumstance occurs and instances where the event or circumstance does not occur.

The substituent "-O-CH₂-O-" means that two oxygen atoms in the substituent are linked to two adjacent carbon atoms in the heterocycloalkyl, aryl or heteroaryl, for example:

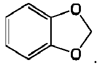

When the number of a linker group is 0, such as -(CH₂)₀-, it means that the linker group is a single bond.

When one of the variables is selected from a chemical bond, it means that the two groups linked by this variable are linked directly. For example, when L in X-L-Y represents a chemical bond, it means that the structure is actually X-Y.

The term "membered ring" includes any cyclic structure. The term "membered" is intended to refer to the number of backbone atoms that form a ring. For example, cyclohexyl, pyridinyl, pyranyl and thiopyranyl are six-membered rings, and cyclopentyl, pyrrolyl, furanyl and thienyl are five-membered rings.

The term "moiety" refers to a specific portion or functional group of a molecule. A chemical moiety is generally considered to be a chemical entity contained in or attached to a molecule.

Unless otherwise stated, the absolute configuration of a stereogenic center is represented by a wedged solid bond ( 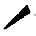 ) and a wedged dashed bond ( 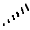 ), and the relative configuration of a stereogenic center is represented by a straight solid bond ( 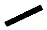 ) and a straight dashed bond ( 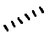 ). A wavy line ( 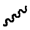 ) represents a wedged solid bond ( 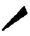 ) or a wedged dashed bond ( 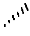 ), or a wavy line ( 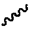 ) represents a straight solid bond ( 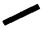 ) or a straight dashed bond ( 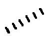 ).

Unless otherwise stated, a single bond or a double bond is represented by -̅ -̅ -̅.

### Specific Pharmaceutical and Medical Terminology

The term "acceptable", as used herein, means that a formulation component or an active ingredient does not unduly and adversely affect a general therapeutic target's health.

The terms "treatment", "treatment course", and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

"Active ingredient" refers to the compound of general formula (1), and pharmaceutically acceptable inorganic or organic salts of the compound of general formula (1). The compound of the present invention may contain one or more asymmetric centers (chiral center or axial chirality) and thus occurs in the forms of a racemate, a racemic mixture, a single enantiomer, a diastereomeric compound and a single diastereomer. Asymmetric centers that may be present depend on the nature of the various substituents on the molecule. Each of such asymmetric centers will independently produce two optical isomers, and all possible optical isomers, diastereomeric mixtures and pure or partially pure compounds are included within the scope of the present invention. The present invention is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present invention are approximations, the related numerical values set forth in the specific examples have been presented herein as precisely as possible. Any numerical value, however, inherently contains a standard deviation necessarily resulting from certain methods of testing. Herein, "about" generally means that the actual value is within a particular value or range ± 10%, 5%, 1%, or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At least, these numerical parameters should be understood as the significant digits indicated or the numerical values obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present invention provides a method for treating a disease, including but not limited to a condition involving ATR protein kinase (e.g., cancer), with the compound of general formula (1) or the pharmaceutical composition of the present invention.

In some embodiments, a method for treating cancer is provided, the method including administering to an individual in need thereof an effective amount of any aforementioned pharmaceutical composition including the compound of structural general formula (1). In some embodiments, the compound of general formula (1) can be used in combination with an additional anti-cancer drug. In some embodiments, the compound of general formula (1) can be used in combination with gemcitabine. In some embodiments, the cancer is mediated by ATR protein kinase. In other embodiments, the cancer is a hematologic cancer and a solid tumor, including but not limited to, leukemia, breast cancer, lung cancer, pancreatic cancer, colon cancer, bladder cancer, brain cancer, urothelial cancer, prostate cancer, liver cancer, ovarian cancer, head and neck cancer, gastric cancer, mesothelioma or all cancer metastases.

### Route of Administration

The compound and the pharmaceutically acceptable salt thereof of the present invention can be made into various formulations including a safe and effective amount of the compound or the pharmaceutically acceptable salt thereof of the present invention, and a pharmaceutically acceptable excipient or carrier, wherein the "safe and effective amount" means that the amount of the compound is sufficient to significantly improve the condition without causing serious adverse effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present invention and with each other, without significantly diminishing the pharmaceutical efficacy of the compound. Examples of pharmaceutically acceptable excipients or carriers include cellulose and derivatives thereof (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets, and pills, the dosage forms may further include buffers.

Solid dosage forms such as tablets, dragées, capsules, pills, and granules can be prepared using coatings and shells such as enteric coatings and other materials well known in the art. They may include opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the excipients described above.

Liquid dosage forms for oral administration include pharmaceutically acceptable emulsions, solutions, suspensions, syrups, and elixirs. In addition to the active compound, the liquid dosage form may include inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, flavoring agents, and perfuming agents.

In addition to the active compound, suspensions may include suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may include physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms for topical administration of the compound of the present invention include ointments, pulvises, patches, sprays, and inhalants. The active ingredient is mixed under sterile conditions with a physiologically acceptable carrier and any preservatives, buffers or propellants that may be required if necessary.

The compound of the present invention may be administered alone or in combination with other pharmaceutically acceptable compounds. When the pharmaceutical composition is used, a safe and effective amount of the compound of the present invention is administered to a mammal (such as a human) to be treated, wherein the dose of administration is a pharmaceutically effective dose. For a human of 60 kg, the daily dose of administration is usually 1-2000 mg, preferably 50-1000 mg. In determining a specific dose, such factors as the route of administration, the health condition of the patient and the like will also be considered, which are well-known to skilled physicians.

The above features mentioned in the present invention or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present invention very clear. It should be understood that the detailed description and examples below describe specific examples for reference only. After reading the description of the present invention, those skilled in the art can make various changes or modifications to the present invention, and such equivalents also fall within the scope of the present application defined herein.

In all the examples, ¹H-NMR spectra were recorded with a Varian Mercury 400 nuclear magnetic resonance spectrometer, and chemical shifts are represented by δ (ppm); silica gel for separation was 200-300 mesh silica gel if not specified, and the ratio of the eluents was a volume ratio.

The following abbreviations are used in the present invention: Ar for argon; AcCl for acetyl chloride; AcOH for glacial acetic acid; acetone for acetone; CDCl₃ for deuterated chloroform; conc HCl for concentrated hydrochloric acid; DIPEA for diisopropylethylamine; DCE for 1,2-dichloroethane; DCM for dichloromethane; dioxane for 1,4-dioxane; DMF for *N*,*N-*dimethylformamide; DMSO for dimethyl sulfoxide; EA or EtOAc for ethyl acetate; h for hour; K₃PO₄ for anhydrous potassium phosphate; KOH for potassium hydroxide; K₂CO₃ for anhydrous potassium carbonate; LC-MS for liquid chromatography-mass spectrometry; MeI for iodomethane; MeOH for anhydrous methanol; mL for milliliter; min for minute; MS for mass spectrometry; NaH for sodium hydride; Na₂SO₄ for sodium sulfate; NaBH₄ for sodium borohydride; NaBH(OAc)₃ for sodium triacetoxyborohydride; NH(CH₃)₂ for dimethylamine; NMR for nuclear magnetic resonance; Pd₂(dba)₃ for tris(dibenzylideneacetone)dipalladium(0); Pd(dtbpf)Cl₂ for dichloro[1,1'-bis(di-*tert*-butylphosphino)ferrocene]palladium(II); PE for petroleum ether; THF for tetrahydrofuran; TFA for trifluoroacetic acid; TfOH for trifluoromethanesulfonic acid; Tf₂O for trifluoromethanesulfonic anhydride; Tol for methylbenzene; prep-HPLC for preparative high performance liquid chromatography; Xantphos for 4,5-bis(diphenylphosphino)-9,9-dimethylxanthene.

### Example 1. Synthesis of (R)-2-(2-(3-methylmorpholino)-8-(1H-pyrazol-5-yl)-1,7-naphthyridin-4-yl)acetonitrile (Compound 1)

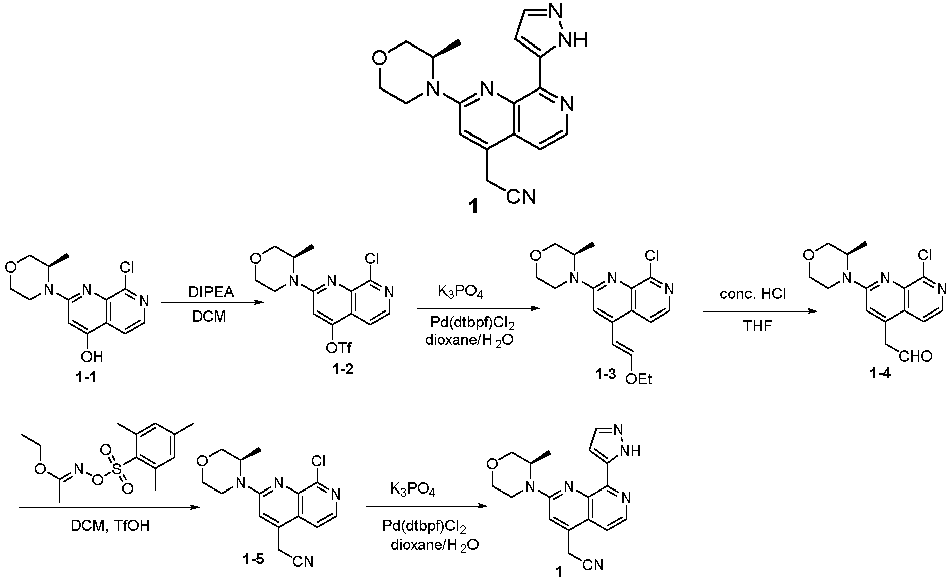

### Step 1: Synthesis of 1-2:

To a 250 mL single-neck flask were added 1-1 (2.4 g, 8.58 mmol), DCM (60 mL) and DIPEA (2.77 g, 21.47 mmol). After the system was purged with Ar, *N-*phenylbis(trifluoromethanesulfonyl)imide (4.6 g, 12.87 mmol) was added, and the mixed solution was stirred at room temperature for 5 h. After the completion of the reaction as detected by LC-MS, water (50 mL) was added to quench the mixed solution, the resulting mixture was stirred, and liquid separation was performed. The aqueous phase was then extracted with DCM (50 mL). The organic phases were combined, washed with saturated sodium chloride solution (50 mL), and concentrated. The residue was purified by column chromatography (EA:PE = 10:0 to 10:1 to 5:1) to give a yellowish oily product (2.7 g, yield: 77.1%), ESI-MS m/z: 412.1 [M+H]⁺.

### Step 2: Synthesis of 1-3:

To a 250 mL single-neck flask were added 1-2 (2.7 g, 6.55 mmol), K₃PO₄ (2.78 g, 13.1 mmol), (E)-2-(2-ethoxyvinyl)-4,4,5,5-tetramethyl-1,3,2-dioxaborolane (1.29 g, 6.55 mmol), Diox (60 mL) and water (12 mL). After the system was purged with Ar, Pd(dtbpf)Cl₂ (427 mg, 0.655 mmol) was added. The mixed solution was purged with argon, heated to 80 °C and stirred for 2.5 h. After the completion of the reaction as detected by LC-MS, the mixed solution was filtered. EA (100 mL) and water (50 mL) were added to the filtrate, the resulting mixture was stirred, and liquid separation was performed. The organic phase was washed with saturated sodium chloride solution (50 mL) and concentrated. The residue was purified by column chromatography (EA:PE = 10:0 to 10:1 to 5:1) to give a yellowish oily product (700 mg, yield: 32.0%), ESI-MS m/z: 334.2 [M+H]⁺.

### Step 3: Synthesis of 1-4:

To a 100 mL single-neck flask were added 1-3 (700 mg, 2.1 mmol), THF (30 mL) and concentrated hydrochloric acid (3 mL). The mixed solution was purged with argon and then stirred at room temperature for 2 h. After the completion of the reaction as detected by LC-MS, DCM (30 mL) was added to the mixed solution, the pH was adjusted to 7-8 with saturated sodium bicarbonate solution, the resulting mixture was stirred, and liquid separation was performed. The aqueous phase was then extracted with DCM (20 mL). The organic phases were combined, washed with saturated sodium chloride solution (20 mL), dried over anhydrous sodium sulfate, filtered and concentrated to give a brownish yellow oily product (730 mg, yield: > 100%), ESI-MS m/z: 306.1/324.1 [M+H]⁺.

### Step 4: Synthesis of 1-5:

To a 100 mL single-neck flask were added 1-4 (730 mg, crude, 2.1 mmol), ethyl (*E*)*-N-*((methylsulfonyl)oxy)acetimidate (1.42 g, 6.0 mmol), and DCM (30 mL). After the system was purged with argon, trifluoromethanesulfonic acid (340 mg, 2.26 mmol) was added, and the mixed solution was stirred at room temperature for 20 h. After the completion of the reaction as detected by LC-MS, saturated sodium bicarbonate solution was added to the mixed solution to adjust the pH to 7-8, the resulting mixture was stirred, and liquid separation was performed. The aqueous phase was then extracted with DCM (20 mL). The organic phases were combined, washed with saturated sodium chloride solution (20 mL) and concentrated. The residue was purified by Flash to give a brownish yellow solid product (400 mg, yield: 62.9%), ESI-MS m/z: 303.1 [M+H]⁺.

### Step 5: Synthesis of compound 1:

To a 100 mL single-neck flask were added 1-5 (50 mg, 0.153 mmol), K₃PO₄ (80 mg, 0.377 mmol), pyrazole-3-boronic acid (30 mg, 0.25 mmol), Diox (5 mL) and water (1 mL). After the system was purged with Ar, Pd(dtbpf)Cl₂ (13 mg, 0.02 mmol) was added. The mixed solution was purged with argon, heated to 80 °C and stirred for 1.5 h. After the completion of the reaction as detected by LC-MS, the mixed solution was filtered and concentrated. The residue was purified by Flash to give a yellowish solid product (10 mg, yield: 19.5%), ESI-MS m/z: 335.1 [M+H]⁺.

¹H NMR (400 MHz, cdcl₃) δ 8.78 (d, *J* = 2.6 Hz, 1H), 8.39 (d, *J* = 5.5 Hz, 1H), 7.86 (d, *J* = 1.6 Hz, 1H), 7.38 (d, *J* = 5.5 Hz, 1H), 7.34 (s, 1H), 6.52 (dd, *J* = 2.6, 1.7 Hz, 1H), 4.44 (s, 1H), 4.14 - 4.05 (m, 4H), 3.86 (d, *J* = 11.5 Hz, 1H), 3.76 (d, *J=* 3.1 Hz, 1H), 3.63 (td, *J* = 12.0, 3.2 Hz, 1H), 3.40 (td, *J* = 12.7, 3.8 Hz, 1H), 1.37 (d, *J=* 6.8 Hz, 3H).

### Examples 2 and 3. Synthesis of 2-(2-((R)-3-methylmorpholino)-8-(1H-pyrazol-5-yl)-1,7-naphthyridin-4-yl)propionitrile (Compound 2) and Synthesis of (R)-2-methyl-2-(2-(3-methylmorpholino)-8-(1H-pyrazol-5-yl)-1,7-naphthyridin-4-yl)propionitrile (Compound 3)

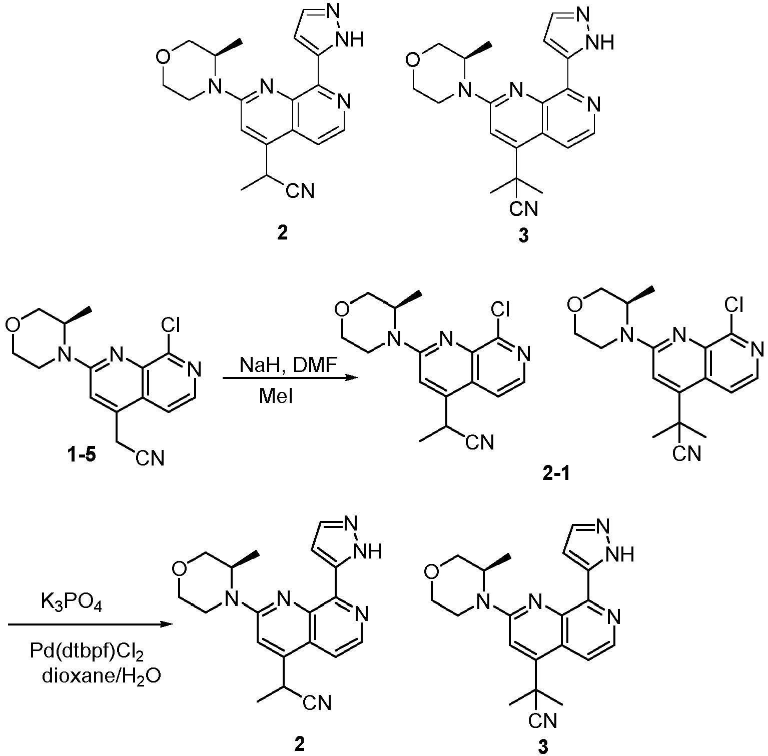

### Step 1: Synthesis of 2-1:

To a 100 mL single-neck flask were added 1-5 (100 mg, 0.331 mmol) and DMF (10 mL). After the system was purged with argon, the mixed solution was cooled to 0-5 °C in an ice bath, and then NaH (26 mg, 60%, 0.662 mmol) was added. The mixed solution was stirred at room temperature for 15 min, and then MeI (70 mg, 0.497 mmol) was added. The mixed solution was stirred at room temperature for 2 h. After the completion of the reaction as detected by LC-MS, the mixed solution was poured into 10 mL of a mixture of ice and water. EA (20 mL) was added, the resulting mixture was stirred, and liquid separation was performed. The aqueous phase was then extracted with EA (10 mL). The organic phases were combined, washed with saturated sodium chloride solution (10 mL), dried over anhydrous sodium sulfate, filtered and concentrated to give a brown oily product (127 mg, yield: > 100%), ESI-MS m/z: 316.2 [M+H]⁺ and 330.2 [M+H]⁺.

### Step 2: Synthesis of compounds 2 and 3:

To a 100 mL single-neck flask were added 2-1 (127 mg, crude, 0.331 mmol), K₃PO₄ (160 mg, 0.754 mmol), pyrazole-3-boronic acid (56 mg, 0.497 mmol), Diox (10 mL) and water (2 mL). After the system was purged with Ar, Pd(dtbpf)Cl₂ (25 mg, 0.038 mmol) was added. The mixed solution was purged with argon, heated to 80 °C and stirred for 1.5 h. After the completion of the reaction as detected by LC-MS, the mixed solution was filtered and concentrated. The residue was purified by Flash to give a yellowish solid mixture. The mixed solution was purified by prep-HPLC to give the compound 2 (10 mg, yield: 8.7%), ESI-MS m/z: 349.2 [M+H]⁺; and the compound 3 (17 mg, yield: 14.2%), ESI-MS m/z: 363.2 [M+H]⁺.

### Examples 4-24. Synthesis of Compounds 4-24

Compounds 4-24 in Table 1 were obtained according to a similar synthetic method to that in Example 2 using different starting materials.

**Table 1**

| **Compound** | **Compound structure** | **[M+H]⁺** | **Compound** | **Compound structure** | **[M+H]⁺** |
|---|---|---|---|---|---|
| **4** | 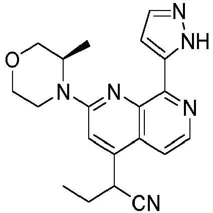 | 363.1 | **5** | 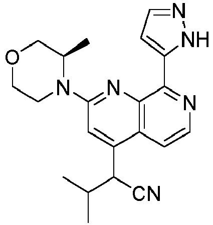 | 377.2 |
| **6** | 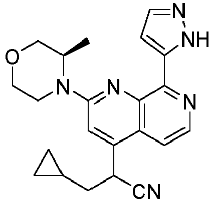 | 389.2 | 7 | 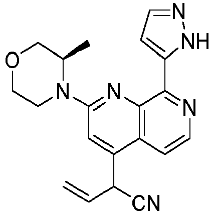 | 361.1 |
| **8** | 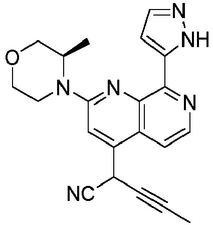 | 373.1 | 9 | 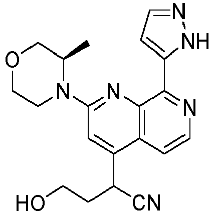 | 379.2 |
| **10** | 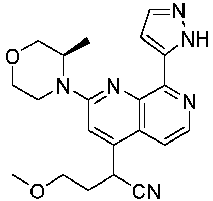 | 393.1 | 11 | 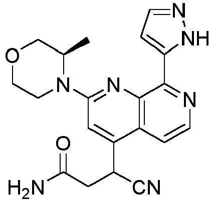 | 392.1 |
| **12** | 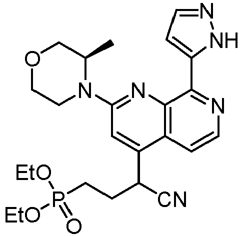 | 499.3 | 13 | 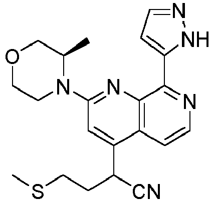 | 409.2 |
| **14** | 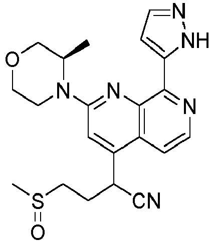 | 425.3 | 15 | 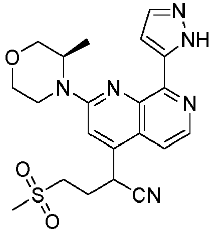 | 441.3 |
| **16** | 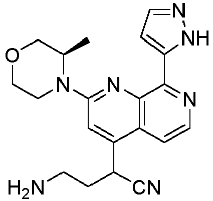 | 378.2 | 17 | 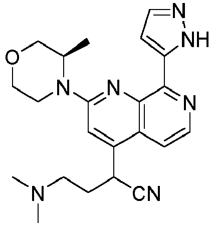 | 406.2 |
| **18** | 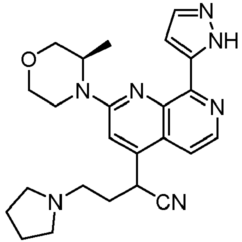 | 432.2 | 19 | 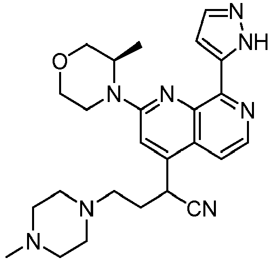 | 461.3 |
| **20** | 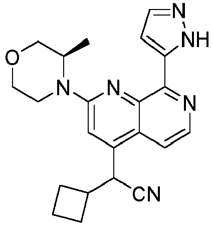 | 389.2 | 21 | 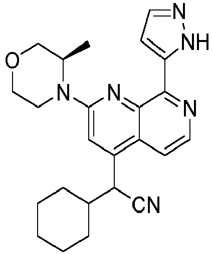 | 417.3 |
| **22** | 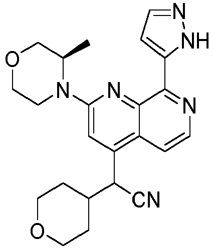 | 419.2 | 23 | 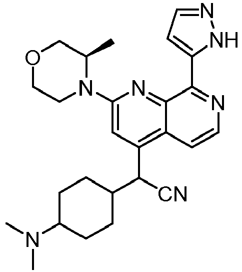 | 460.3 |
| **24** | 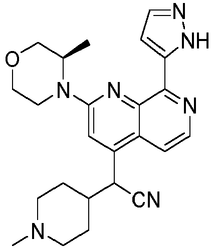 | 432.2 | | | |

### Example 25. Synthesis of (R)-1-(2-(3-methylmorpholino)-8-(1H-pyrazol-5-yl)-1,7-naphthyridin-4-ylmethyl)cyclohexane-1-carbonitrile (Compound 25)

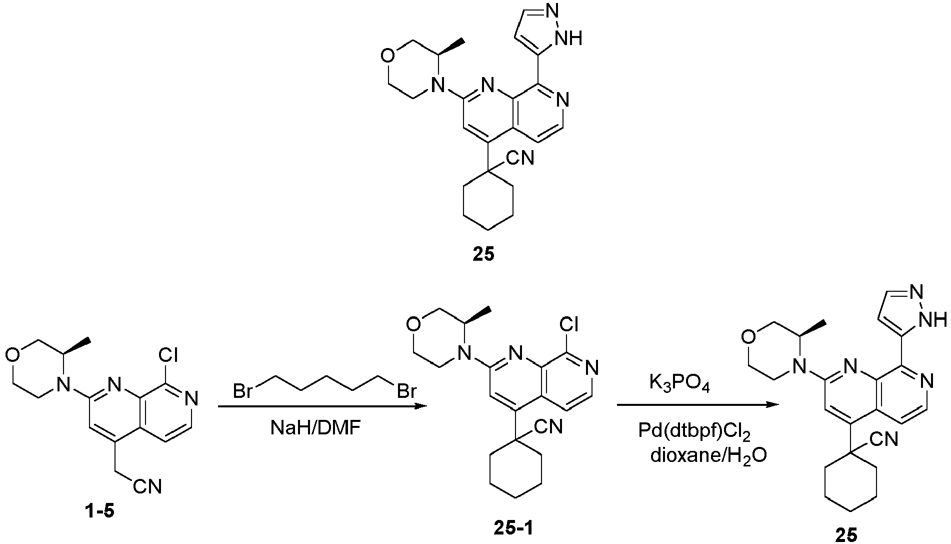

### Step 1: Synthesis of 25-1:

To a 100 mL single-neck flask were added 1-5 (100 mg, 0.331 mmol) and DMF (10 mL). After the system was purged with argon, the mixed solution was cooled to 0-5 °C in an ice bath, and then NaH (26 mg, 60%, 0.662 mmol) was added. The mixed solution was stirred at room temperature for 15 min, and then 1,5-dibromohexane (76 mg, 0.331 mmol) was added. The mixed solution was stirred at room temperature for 2 h. After the completion of the reaction as detected by LC-MS, the mixed solution was poured into 10 mL of a mixture of ice and water. EA (20 mL) was added, the resulting mixture was stirred, and liquid separation was performed. The aqueous phase was then extracted with EA (10 mL). The organic phases were combined, washed with saturated sodium chloride solution (10 mL), dried over anhydrous sodium sulfate, filtered and concentrated to give a brown oily product (140 mg, yield: > 100%), ESI-MS m/z: 371.2 [M+H]⁺.

### Step 2: Synthesis of compound 25:

To a 100 mL single-neck flask were added 25-1 (140 mg, crude, 0.331 mmol), K₃PO₄ (160 mg, 0.754 mmol), pyrazole-3-boronic acid (56 mg, 0.497 mmol), dioxane (10 mL) and water (2 mL). After the system was purged with Ar, Pd(dtbpf)Cl₂ (25 mg, 0.038 mmol) was added. The mixed solution was purged with argon, heated to 80 °C and stirred for 1.5 h. After the completion of the reaction as detected by LC-MS, the mixed solution was filtered and concentrated. The residue was purified by Flash to give a yellowish solid product (46 mg, yield: 34.5%), ESI-MS m/z: 403.3 [M+H]⁺.

¹H NMR (400 MHz, CDCl₃) δ 8.48 (d, *J* = 5.7 Hz, 1H), 8.04 (d, *J* = 5.7 Hz, 1H), 7.70 (d, *J* = 1.8 Hz, 1H), 7.31 (d, *J* = 1.8 Hz, 1H), 7.21 (s, 1H), 4.42 (d, *J* = 5.0 Hz, 1H), 4.17 (dd, *J* = 11.5, 3.8 Hz, 1H), 4.00 - 3.94 (m, 1H), 3.91 (s, 1H), 3.85 (dd, *J* = 11.6, 3.1 Hz, 1H), 3.71 (td, *J* = 11.8, 3.0 Hz, 1H), 3.55 (td, *J* = 12.4, 3.9 Hz, 1H), 2.55 (d, *J* = 12.7 Hz, 2H), 2.02 (d, *J* = 11.4 Hz, 6H), 1.85 (dd, *J* = 12.7, 4.1 Hz, 2H), 1.44 (d, *J* = 6.8 Hz, 3H).

### Examples 26-32. Synthesis of Compounds 26-32

Compounds 26-32 in Table 2 were obtained according to a similar synthetic method to that in Example 25 using different starting materials.

**Table 2**

| **Compound** | **Compound structure** | **[M+H]⁺** | **Compound** | **Compound structure** | **[M+H]⁺** |
|---|---|---|---|---|---|
| **26** | 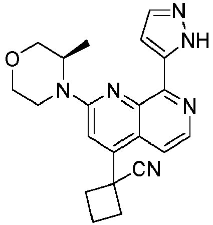 | 375.2 | **27** | 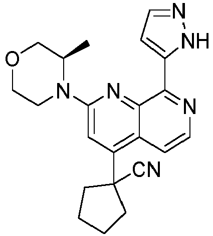 | 389.2 |
| **28** | 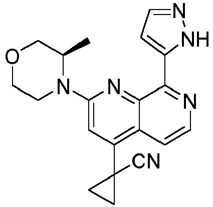 | 361.2 | **29** | 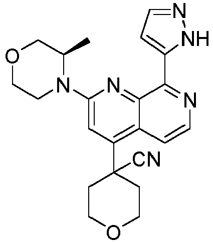 | 405.2 |
| **30** | 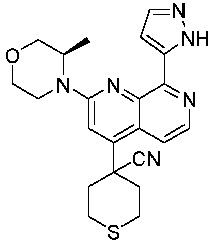 | 421.2 | **31** | 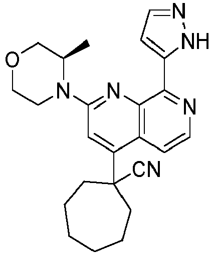 | 417.2 |
| **32** | 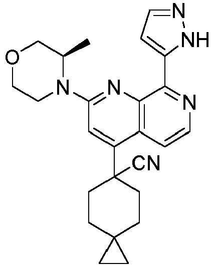 | 429.2 | | | |

### Example 33. Synthesis of (R)-4-hydroxy-1-(2-(3-methylmorpholine)-8-(1H-pyrazol-5-yl)-1,7-naphthyridin-4-yl)cyclohexane-1-carbonitrile (Compound 33)

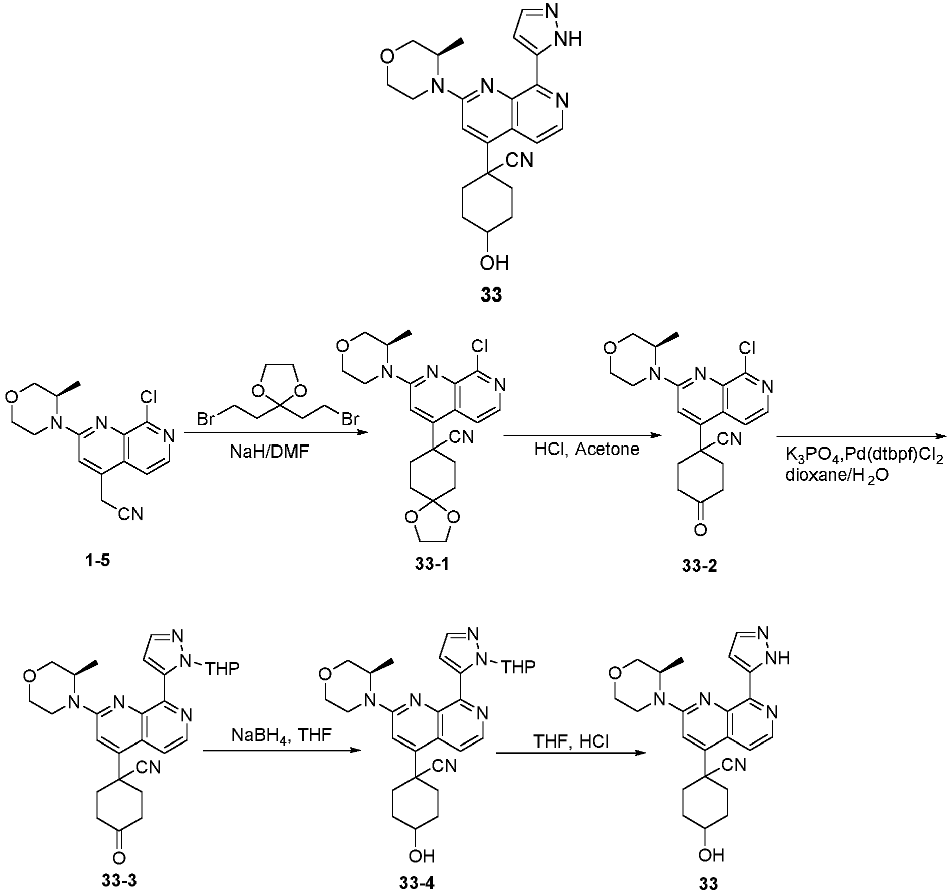

### Step 1: Synthesis of 33-1:

To a 500 mL single-neck flask were added 1-5 (2.0 g, 6.6 mmol) and DMF (200 mL). After the system was purged with argon, the mixed solution was cooled to 0-5 °C in an ice bath, and then NaH (528 mg, 60%, 13.2 mmol) was added. The mixed solution was stirred at room temperature for 30 min, and then 2,2-bis(2-bromoethyl)-1,3-dioxolane (2.85 g, 9.9 mmol) was added. The mixed solution was stirred at room temperature for 3 h. After the completion of the reaction as detected by LC-MS, the mixed solution was poured into 200 mL of a mixture of ice and water. EA (200 mL) was added, the resulting mixture was stirred, and liquid separation was performed. The aqueous phase was then extracted with EA (100 mL). The organic phases were combined, washed with saturated sodium chloride solution (200 mL), dried over anhydrous sodium sulfate, filtered and concentrated to give a brown oily product (2.12 g, yield: 75%), ESI-MS m/z: 429.1 [M+H]⁺.

### Step 2: Synthesis of 33-2:

To a 500 mL single-neck flask were added 33-1 (2.12 g, 4.94 mmol), acetone (50 mL) and 0.5 N HCl (20 mL). The mixed solution was purged with Ar, heated to 50 °C and stirred for 5 h. After the completion of the reaction as detected by LC-MS, the mixed solution was concentrated to half volume, EA (100 mL) was added, the pH was adjusted to 7-8 with saturated sodium bicarbonate solution, the resulting mixture was stirred, and liquid separation was performed. The aqueous phase was then extracted with EA (50 mL). The organic phases were combined, washed with saturated sodium chloride solution, dried over anhydrous sodium sulfate, filtered and concentrated to dryness to give a brown oily product (1.81 g, yield: 95%), ESI-MS m/z: 385.0 [M+H]⁺.

### Step 3: Synthesis of 33-3:

To a 500 mL single-neck flask were added 33-2 (1.81 g, 4.7 mmol), K₃PO₄ (2.99 g, 14.1 mmol), (1-(tetrahydro-2*H*-pyran-2-yl)-1*H*-pyrazol-5-yl)boronic acid (1.38 g, 7.05 mmol), dioxane (160 mL) and water (32 mL). After the system was purged with Ar, Pd(dtbpf)Cl₂ (309 mg, 0.47 mmol) was added. The mixed solution was purged with argon, heated to 80 °C and stirred for 2 h. After the completion of the reaction as detected by LC-MS, the mixed solution was filtered and concentrated. The residue was purified by Flash to give a yellowish solid product (1.67 g, yield: 71%), ESI-MS m/z: 501.3 [M+H]⁺.

### Step 4: Synthesis of 33-4:

To a 100 mL single-neck flask were added 33-3 (100 mg, 0.2 mmol), THF (10 mL), MeOH (5 mL) and NaBH₄ (6 mg, 0.16 mmol). The mixed solution was stirred at room temperature for 2 h. After the completion of the reaction as detected by LC-MS, water (2 mL) was added to quench the mixed solution, and the resulting mixture was then concentrated under reduced pressure to give a crude product, ESI-MS m/z: 503.3 [M+H]⁺.

### Step 5: Synthesis of 33:

To a 100 mL single-neck flask were added the crude 33-4 described above, THF (10 mL) and 2 N HCl (3 mL). The mixed solution was purged with Ar and then stirred at room temperature for 2 h. After the completion of the reaction as detected by LC-MS, the mixed solution was concentrated under reduced pressure. The residual liquid was purified by Flash to give a product (8 mg, yield: 9.5%), ESI-MS m/z: 419.3 [M+H]⁺.

¹H NMR (400 MHz, cdcl₃) δ 8.50 (d, *J=* 5.8 Hz, 1H), 8.00 (d, *J=* 5.8 Hz, 1H), 7.71 (d, *J* = 1.9 Hz, 1H), 7.32 (d, *J =* 1.9 Hz, 1H), 7.20 (s, 1H), 4.42 (d, *J* = 7.0 Hz, 1H), 4.18 (dd, *J* = 11.4, 3.8 Hz, 1H), 4.01 - 3.95 (m, 1H), 3.93 (d, *J* = 11.4 Hz, 1H), 3.85 (dd, *J* = 11.5, 3.1 Hz, 1H), 3.80 - 3.65 (m, 2H), 3.55 (td, *J* = 12.4, 3.8 Hz, 1H), 2.62 (d, *J =* 12.2 Hz, 2H), 2.27 (d, *J =* 12.9 Hz, 2H), 2.12 - 1.92 (m, 4H), 1.44 (s, 3H).

### Example 34. Synthesis of (R)-4-(dimethylamino)-1-(2-(3-methylmorpholine)-8-(1H-pyrazol-5-yl)-1,7-naphthyridin-4-yl)cyclohexane-1-carbonitrile (Compound 34)

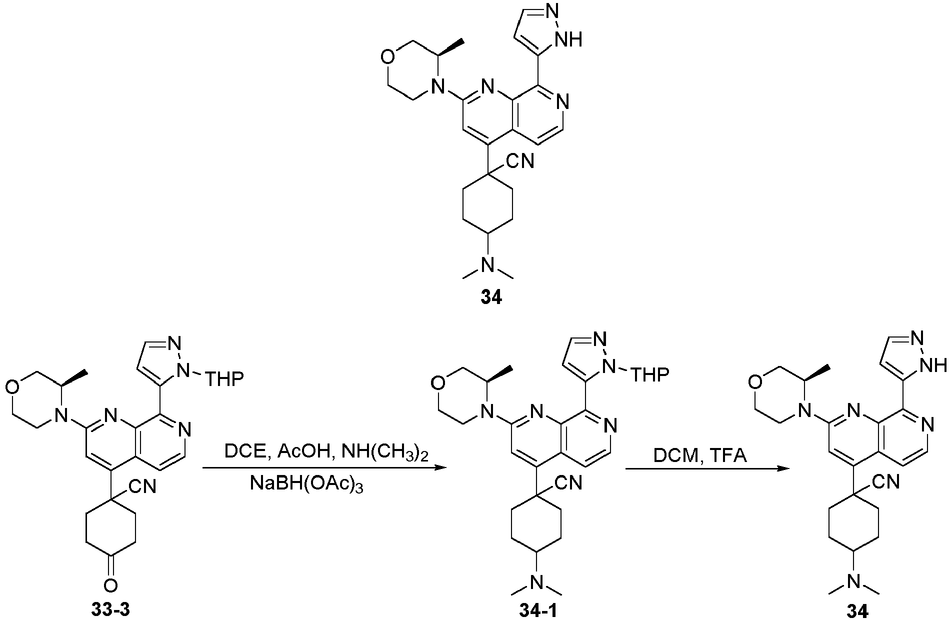

### Step 1: Synthesis of 34-1:

To a 100 mL single-neck flask were added 33-3 (100 mg, 0.205 mmol), DCE (10 mL), AcOH (38 mg, 0.63 mmol) and dimethylamine (0.5 mL, 2 M in THF, 1.0 mmol). The mixed solution was stirred at room temperature for 30 min, and then NaBH(OAc)₃ (87 mg, 0.41 mmol) was added. The mixed solution was heated to 50 °C and stirred for 3 h. After the reaction was substantially completed as detected by LC-MS, the mixed solution was concentrated. The residue was purified by Flash to give a product (32 mg, yield: 29.5%), ESI-MS m/z: 530.3 [M+H]⁺.

### Step 2: Synthesis of 34:

To a 100 mL single-neck flask were added 34-1 (32 mg, 0.06 mmol), DCM (10 mL) and TFA (0.2 mL). The mixed solution was purged with Ar and then stirred at room temperature for 2 h. After the completion of the reaction as detected by LC-MS, the mixed solution was concentrated under reduced pressure. The residual liquid was purified by Flash to give a product (12 mg, yield: 44.9%), ESI-MS m/z: 446.3 [M+H]⁺.

¹H NMR (400 MHz, Chloroform-*d*) δ 8.49 (d, *J=* 5.7 Hz, 1H), 8.01 (d, *J=* 5.7 Hz, 1H), 7.70 (d, *J* = 1.8 Hz, 1H), 7.31 (d, *J=* 1.9 Hz, 1H), 7.20 (s, 1H), 4.46 - 4.38 (m, 1H), 4.17 (dd, *J=* 11.4, 3.8 Hz, 1H), 3.97 (dd, *J* = 12.5, 2.9 Hz, 1H), 3.92 (d, *J* = 11.4 Hz, 1H), 3.84 (dd, *J* = 11.5, 2.9 Hz, 1H), 3.70 (td, *J* = 11.8, 3.0 Hz, 1H), 3.55 (td, *J* = 12.4, 3.9 Hz, 1H), 2.65 (d, *J* = 12.7 Hz, 2H), 2.44 (d, *J=* 11.2 Hz, 1H), 2.39 (s, 6H), 2.15 (d, *J=* 13.0 Hz, 2H), 2.09 - 1.99 (m, 2H), 1.93 (t, *J=* 12.0 Hz, 2H), 1.44 (d, *J=* 6.8 Hz, 3H).

### Examples 35-36. Synthesis of Compounds 35-36

Compounds 35-36 in Table 3 were obtained according to a similar synthetic method to that in Example 34 using different starting materials.

**Table 3**

| **Compound** | **Compound structure** | **[M+H]⁺** | **Compound** | **Compound structure** | **[M+H]⁺** |
|---|---|---|---|---|---|
| **35** | 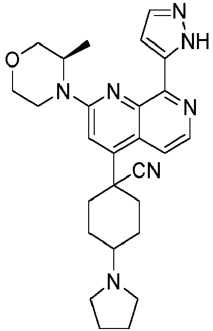 | 472.3 | **36** | 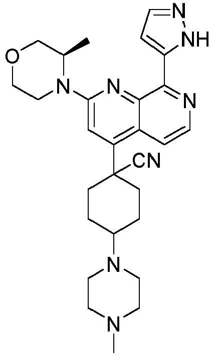 | 501.3 |

### Example 37. Synthesis of (R)-4-(2-(3-methylmorpholino)-8-(1H-pyrazol-5-yl)-1,7-naphthyridin-4-yl)piperidine-4-carbonitrile (Compound 37)

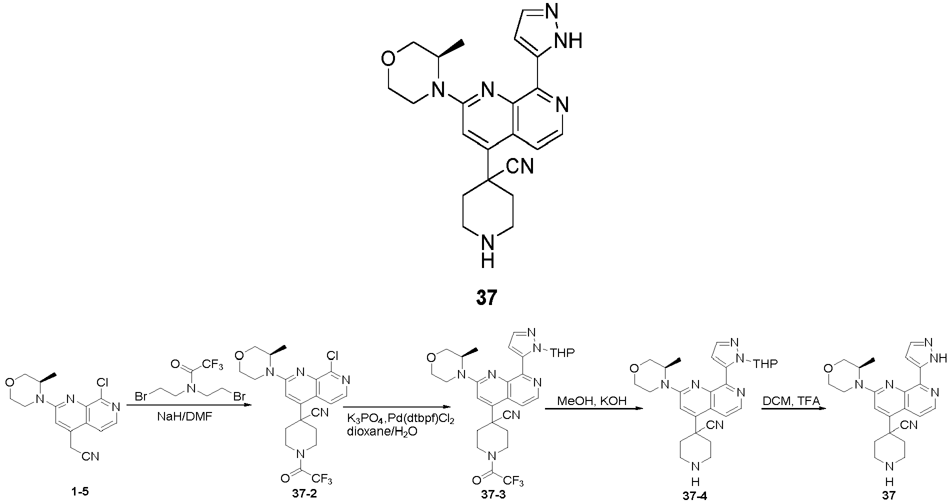

### Step 1: Synthesis of 37-2:

To a 500 mL single-neck flask were added 1-5 (2.0 g, 6.6 mmol) and DMF (200 mL). After the system was purged with argon, the mixed solution was cooled to 0-5 °C in an ice bath, and then NaH (528 mg, 60%, 13.2 mmol) was added. The mixed solution was stirred at room temperature for 30 min, and then N,N-bis(2-bromoethyl)-2,2,2-trifluoroacetamide (3.24 g, 9.9 mmol) was added. The mixed solution was stirred at room temperature for 2 h. After the completion of the reaction as detected by LC-MS, the mixed solution was poured into 200 mL of a mixture of ice and water. EA (200 mL) was added, the resulting mixture was stirred, and liquid separation was performed. The aqueous phase was then extracted with EA (100 mL). The organic phases were combined, washed with saturated sodium chloride solution (200 mL), dried over anhydrous sodium sulfate, filtered and concentrated to give a brown oily product (1.61 g, yield: 52%), ESI-MS m/z: 467.3 [M+H]⁺.

### Step 2: Synthesis of 37-3:

To a 500 mL single-neck flask were added 37-2 (1.61 g, crude, 3.44 mmol), K₃PO₄ (1.66 g, 7.84 mmol), (1-(tetrahydro-2*H*-pyran-2-yl)-1*H*-pyrazol-5-yl)boronic acid (1.01 g, 5.164 mmol), dioxane (160 mL) and water (32 mL). After the system was purged with Ar, Pd(dtbpf)Cl₂ (250 mg, 0.38 mmol) was added. The mixed solution was purged with argon, heated to 80 °C and stirred for 2 h. After the completion of the reaction as detected by LC-MS, the mixed solution was filtered and concentrated. The residue was purified by Flash to give a yellowish solid product (1.63 g, yield: 81%), ESI-MS m/z: 584.3 [M+H]⁺.

### Step 3: Synthesis of 37-4:

To a 500 mL single-neck flask were added 37-3 (1.60 g, 2.742 mmol), MeOH (50 mL) and 1 N KOH (4 mL, 4 mmol). The mixed solution was purged with Ar and then stirred at room temperature for 20 h. After the completion of the reaction as detected by LC-MS, 1 N HCl (2 mL) was added to the mixed solution, and the resulting mixture was concentrated under reduced pressure to a small amount. The residual liquid was purified by Flash to give a yellowish solid product (1.0 g, yield: 75%), ESI-MS m/z: 488.3 [M+H]⁺.

### Step 4: Synthesis of 37:

To a 100 mL single-neck flask were added 37-4 (50 mg, 0.103 mmol), DCM (5 mL) and TFA (0.2 mL). The mixed solution was purged with Ar and then stirred at room temperature for 2 h. After the completion of the reaction as detected by LC-MS, the mixed solution was concentrated under reduced pressure. The residual liquid was purified by Flash to give a product (11 mg, yield: 26.6%), ESI-MS m/z: 404.2 [M+H]⁺.

### Example 38. Synthesis of (R)-1-acetyl-4-(2-(3-methylmorpholino)-8-(1H-pyrazol-5-yl)-1,7-naphthyridin-4-yl)piperidine-4-carbonitrile (Compound 38)

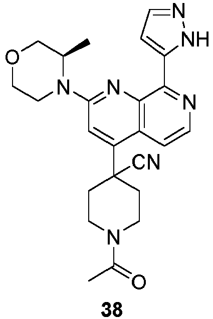

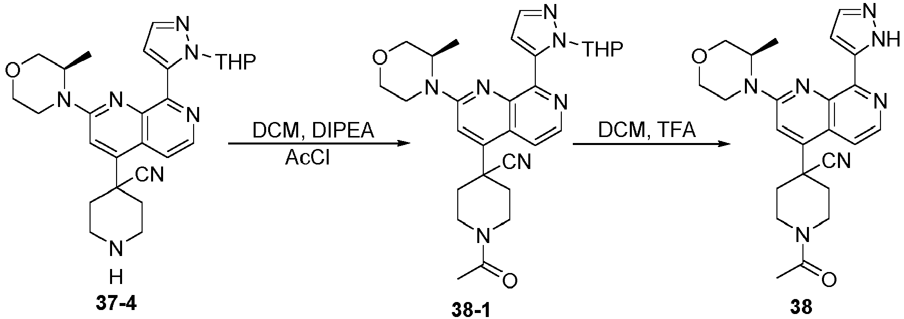

### Step 1: Synthesis of 38-1:

To a 100 mL single-neck flask were added 37-4 (100 mg, 0.205 mmol), DCM (10 mL) and DIPEA(79 mg, 0.615 mmol). After the system was purged with Ar, acetyl chloride (24 mg, 0.306 mmol) was added dropwise. After the addition, the mixed solution was stirred at room temperature for 2 h. After the reaction was substantially completed as detected by LC-MS, water (10 mL) was added to the mixed solution, and liquid separation was performed. The aqueous phase was then extracted with DCM (10 mL). The organic phases were combined, washed with saturated sodium chloride solution (10 mL), dried over anhydrous sodium sulfate, filtered and concentrated to give a product (120 mg, yield: > 100%), ESI-MS m/z: 530.3 [M+H]⁺.

### Step 2: Synthesis of 38:

To a 100 mL single-neck flask were added 38-1 (120 mg, crude, 0.205 mmol), DCM (10 mL) and TFA (0.5 mL). The mixed solution was purged with Ar and then stirred at room temperature for 2 h. After the completion of the reaction as detected by LC-MS, the mixed solution was concentrated under reduced pressure. The residual liquid was purified by Flash to give a product (45 mg, yield: 49.3%), ESI-MS m/z: 446.2 [M+H]⁺.

### Examples 39-40. Synthesis of Compounds 39-40

Compounds 39-40 in Table 4 were obtained according to a similar synthetic method to those in Examples 37 and 38 using different starting materials.

**Table 4**

| **Compound** | **Compound structure** | **[M+H]⁺** | **Compound** | **Compound structure** | **[M+H]⁺** |
|---|---|---|---|---|---|
| **39** | 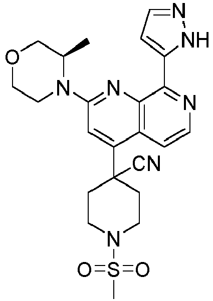 | 482.3 | **40** | 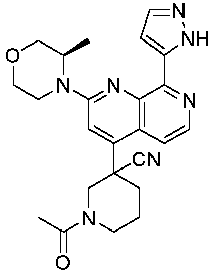 | 446.2 |

### Example 41. Synthesis of (R)-4-(2-(3-methylmorpholino)-8-(1H-pyrazol-5-yl)-1,7-naphthyridin-4-yl)-1-(2,2,2-trifluoroethyl)piperidine-4-carbonitrile (Compound 41)

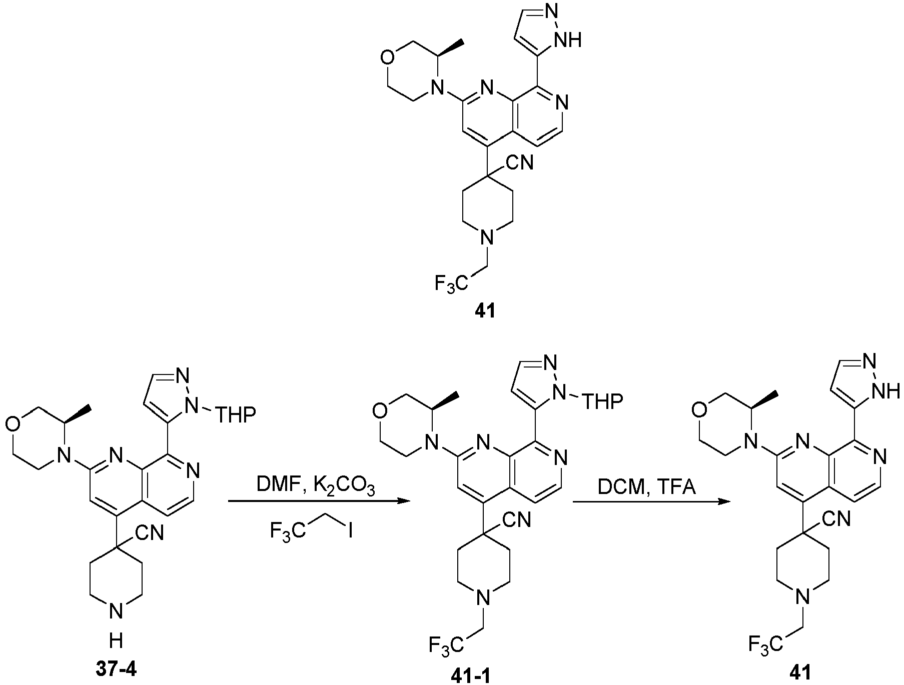

### Step 1: Synthesis of 41-1:

To a 100 mL single-neck flask were added 37-4 (100 mg, 0.205 mmol), DMF (5 mL) and K₂CO₃ (85 mg, 0.615 mmol). After the system was purged with Ar, trifluoroiodoethane (65 mg, 0.31 mmol) was added, and the mixed solution was stirred at room temperature for 20 h. After the completion of the reaction as detected by LC-MS, EA (20 mL) and water (10 mL) were added to the mixed solution, the resulting mixture was stirred, and liquid separation was performed. The aqueous phase was then extracted with EA (10 mL). The organic phases were combined, washed with saturated sodium chloride solution (10 mL), dried over anhydrous sodium sulfate, filtered and concentrated under reduced pressure to give a brown oily product (201 mg, yield: > 100%, containing DMF), ESI-MS m/z: 570.3 [M+H]⁺.

### Step 2: Synthesis of 41:

To a 100 mL single-neck flask were added 41-1 (201 mg, 0.205 mmol, crude), DCM (10 mL) and TFA (0.5 mL). The mixed solution was purged with Ar and then stirred at room temperature for 2 h. After the completion of the reaction as detected by LC-MS, the mixed solution was concentrated under reduced pressure. The residual liquid was purified by Flash to give a product (16 mg, yield: 16.1%), ESI-MS m/z: 486.2 [M+H]⁺.

### Examples 42-44. Synthesis of Compounds 42-44

Compounds 42-44 in Table 5 were obtained according to a similar synthetic method to that in Example 41 using different starting materials.

**Table 5**

| **Compound** | **Compound structure** | **[M+H]⁺** | **Compound** | **Compound structure** | **[M+H]⁺** |
|---|---|---|---|---|---|
| **42** | 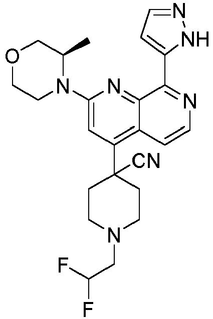 | 468.3 | **43** | 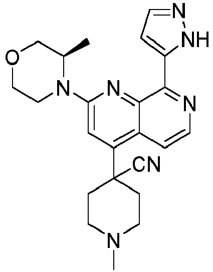 | 418.2 |
| **44** | 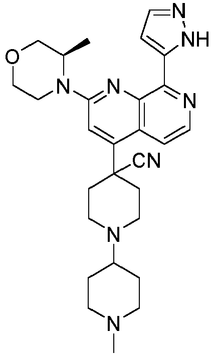 | 501.3 | | | |

### Example 45. Synthesis of (R)-1-(4-chlorophenyl)-4-(2-(3-methylmorpholino)-8-(1H-pyrazol-5-yl)-1,7-naphthyridin-4-yl)piperidine-4-carbonitrile (Compound 45)

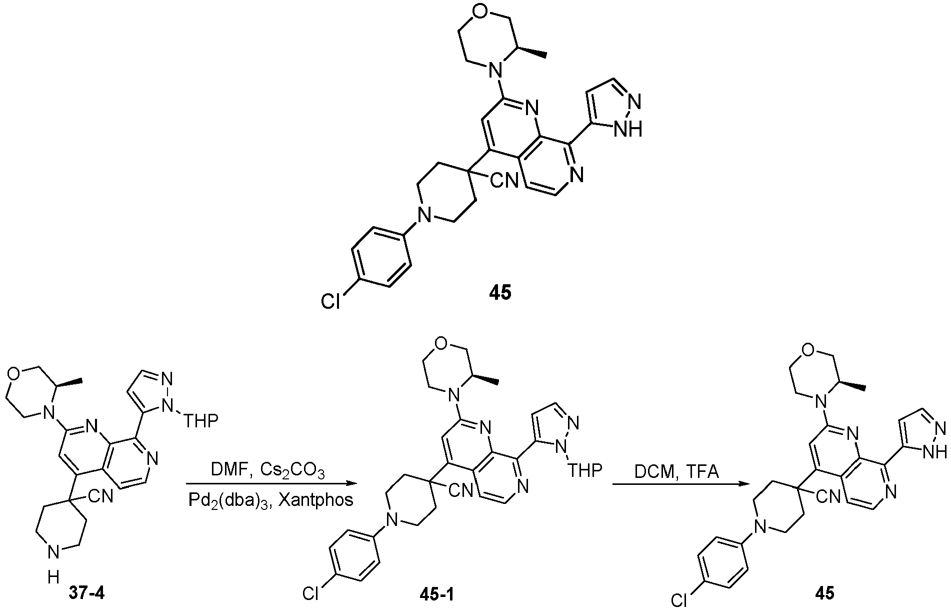

### Step 1: Synthesis of 45-1:

To a 100 mL single-neck flask were added 37-4 (100 mg, 0.205 mmol), DMF (5 mL), Cs₂CO₃ (200 mg, 0.615 mmol), Xantphos (24 mg, 0.041 mmol), 4-chloroiodobenzene (98 mg, 0.41 mmol) and Pd₂(dba)₃ (19 mg, 0.021 mmol). The mixed solution was purged with Ar, heated to 120 °C and stirred for 5 h. After the reaction was substantially completed as detected by LC-MS, the mixed solution was filtered. The filtrate was purified by Flash to give a product (35 mg, yield: 28.5%), ESI-MS m/z: 598.1 [M+H]⁺.

### Step 2: Synthesis of 45:

To a 100 mL single-neck flask were added 45-1 (35 mg, 0.059 mmol), DCM (10 mL) and TFA (0.2 mL). The mixed solution was purged with Ar and then stirred at room temperature for 2 h. After the completion of the reaction as detected by LC-MS, the mixed solution was concentrated under reduced pressure. The residual liquid was purified by Flash to give a product (19 mg, yield: 63.2%), ESI-MS m/z: 514.1 [M+H]⁺.

### Examples 46-50. Synthesis of Compounds 46-50

Compounds 46-50 in Table 6 were obtained according to a similar synthetic method to that in Example 45 using different starting materials.

**Table 6**

| **Compound** | **Compound structure** | **[M+H]⁺** | **Compound** | **Compound structure** | **[M+H]⁺** |
|---|---|---|---|---|---|
| **46** | 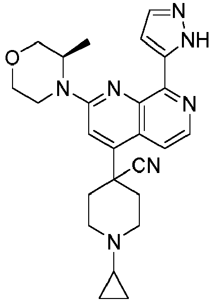 | 444.3 | **47** | 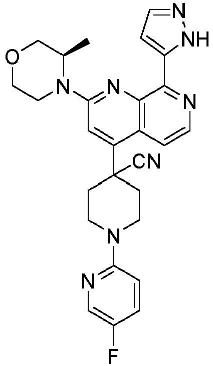 | 499.3 |
| **48** | 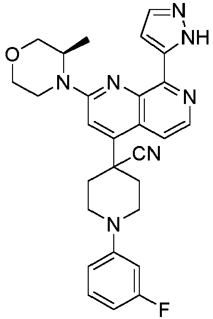 | 497.3 | **49** | 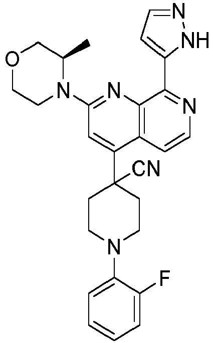 | 497.3 |
| **50** | 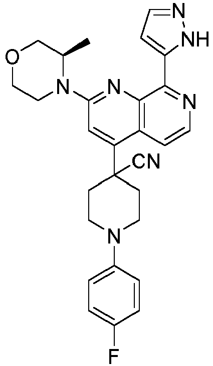 | 497.3 | | | |

### Examples 51-52. Synthesis of Compounds 51-52

Compounds 51-52 in Table 7 were obtained according to a similar synthetic method to that in Example 25 using different starting materials.

**Table 7**

| **Compound** | **Compound structure** | **[M+H]⁺** | **Compound** | **Compound structure** | **[M+H]⁺** |
|---|---|---|---|---|---|
| **51** | 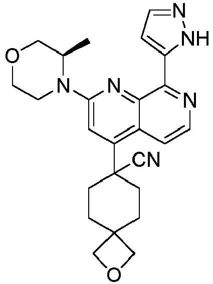 | 445.2 | **52** | 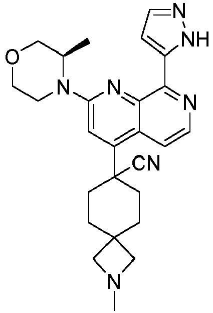 | 458.2 |

### Example 53. Synthesis of (R)-4-methoxy-1-(2-(3-methylmorpholine)-8-(1H-pyrazol-5-yl)-1,7-naphthyridin-4-yl)cyclohexane-1-carbonitrile (Compound 53)

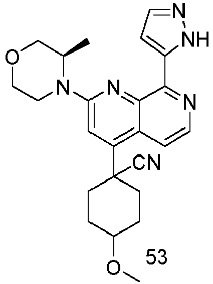

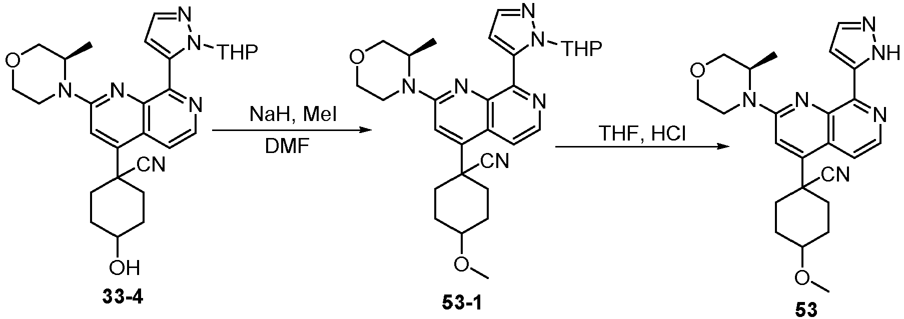

### Step 1: Synthesis of 53-1:

To a 50 mL single-neck flask were added 33-4 (52 mg, 0.103 mmol) and DMF (5 mL). After the system was purged with Ar, NaH (6.2 mg, 60% in mineral oil, 0.155 mmol) was added. The mixed solution was stirred at room temperature for 10 min, and then MeI (22 mg, 0.155 mmol) was added. The mixed solution was stirred at room temperature for 2 h. After the reaction was substantially completed as detected by LC-MS, the mixed solution was directly used in the next step.

### Step 2: Synthesis of 53:

THF (2 mL) and 1 N HCl solution (2 mL, 2 mmol) were added to the reaction mixture described above, and the mixed solution was stirred at room temperature for 2 h. After the completion of the reaction as detected by LC-MS, the mixed solution was purified by Flash to give a product (16 mg, yield: 35.9%), ESI-MS m/z: 433.2 [M+H]⁺.

### Examples 54-59. Synthesis of Compounds 54-59

Compounds 54-59 in Table 8 were obtained according to a similar synthetic method to that in Example 53 using different starting materials.

**Table 8**

| **Compound** | **Compound structure** | **[M+H]⁺** | **Compound** | **Compound structure** | **[M+H]⁺** |
|---|---|---|---|---|---|
| **54** | 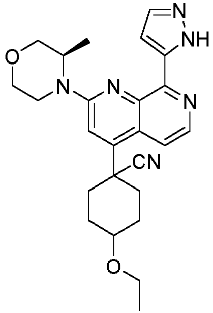 | 447.2 | **55** | 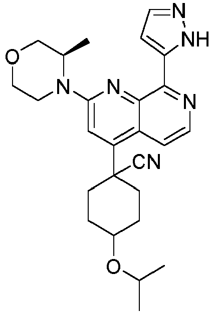 | 461.2 |
| **56** | 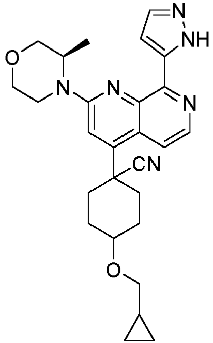 | 473.3 | **57** | 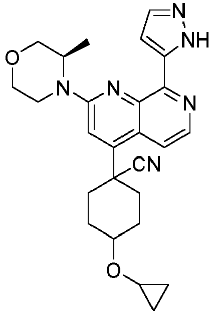 | 459.2 |
| **58** | 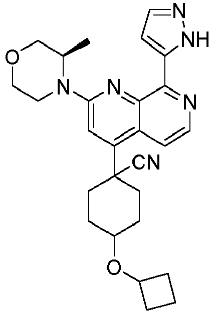 | 473.2 | **59** | 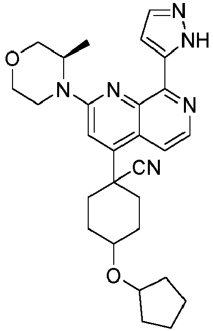 | 487.3 |

### Examples 60-65. Synthesis of Compounds 60-65

Compounds 60-65 in Table 9 were obtained according to a similar synthetic method to that in Example 34 using different starting materials.

**Table 9**

| **Compound** | **Compound structure** | **[M+H]⁺** | **Compound** | **Compound structure** | **[M+H]⁺** |
|---|---|---|---|---|---|
| **60** | 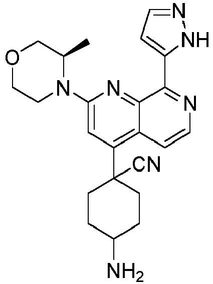 | 418.2 | **61** | 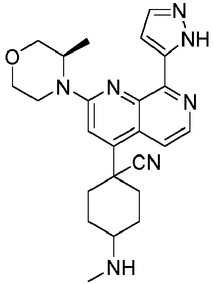 | 432.2 |
| **62** | 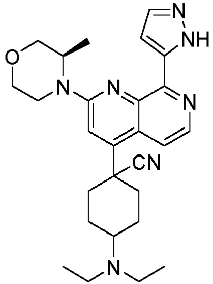 | 474.3 | **63** | 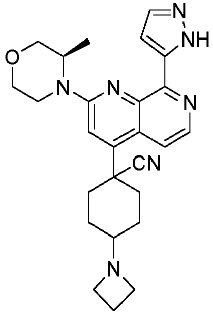 | 458.2 |
| **64** | 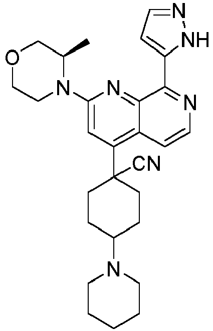 | 486.3 | **65** | 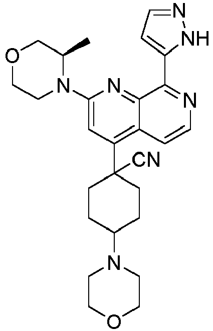 | 488.3 |

### Examples 66-84. Synthesis of Compounds 66-84

Compounds 66-84 in Table 10 were obtained according to a similar synthetic method to that in Example 41 using different starting materials.

**Table 10**

| **Compound** | **Compound structure** | **[M+H]⁺** | **Compound** | **Compound structure** | **[M+H]⁺** |
|---|---|---|---|---|---|
| **66** | 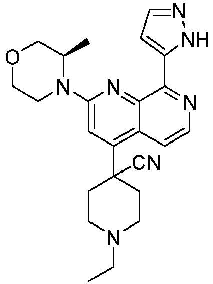 | 432.2 | **67** | 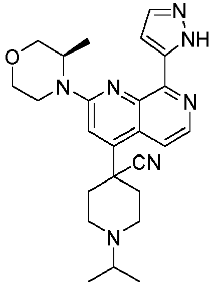 | 446.2 |
| **68** | 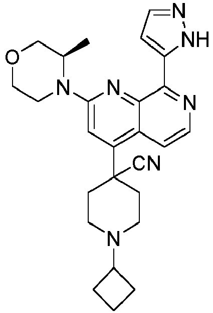 | 458.2 | **69** | 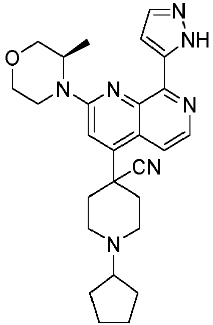 | 472.3 |
| **70** | 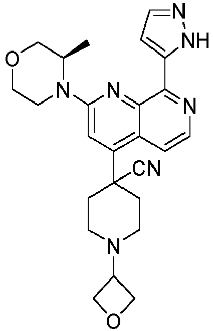 | 460.2 | **71** | 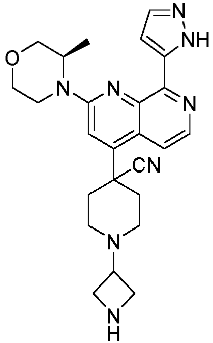 | 459.2 |
| **72** | 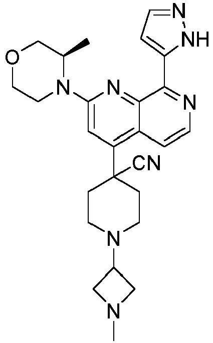 | 473.2 | **73** | 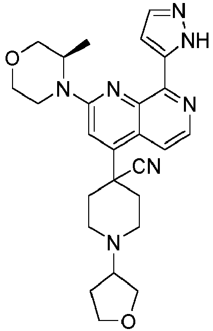 | 474.2 |
| **74** | 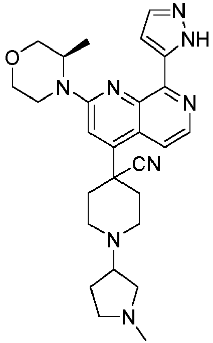 | 487.3 | **75** | 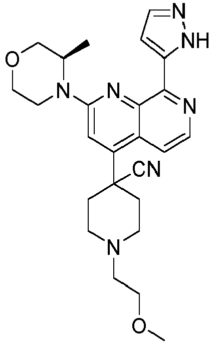 | 462.2 |
| **76** | 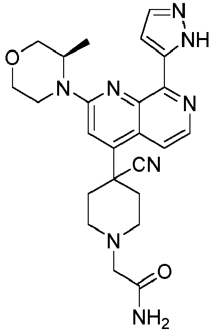 | 461.2 | **77** | 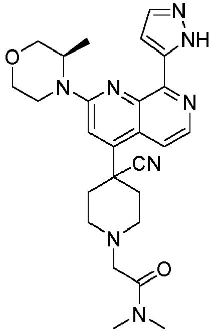 | 489.2 |
| **78** | 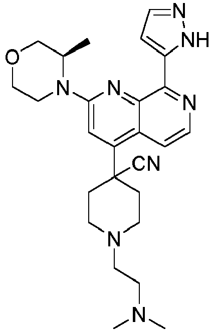 | 475.3 | **79** | 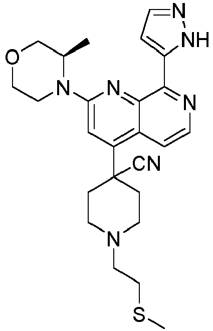 | 478.3 |
| **80** | 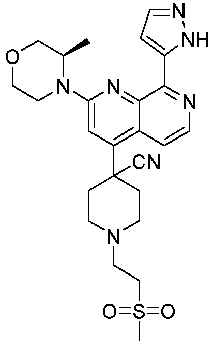 | 509.3 | **81** | 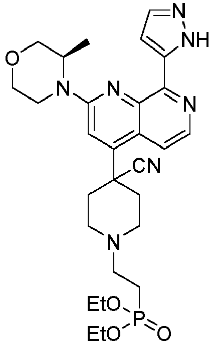 | 568.3 |
| **82** | 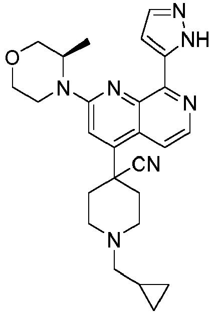 | 458.2 | **83** | 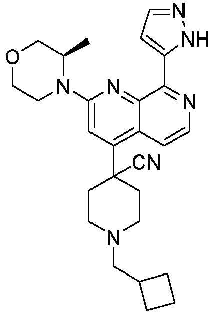 | 472.3 |
| **84** | 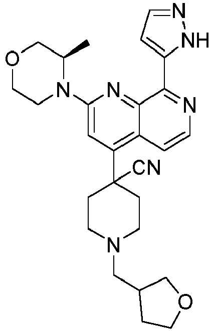 | 488.3 | | | |

### Example 85. Synthesis of 3-(2-((R)-3-methylmorpholino)-8-(1H-pyrazol-5-yl)-1,7-naphthyridin-4-yl)-8-oxabicyclo[3.2.1]octane-3-carbonitrile (Compound 85)

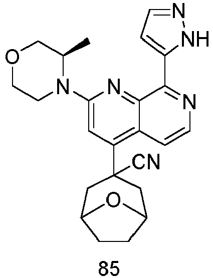

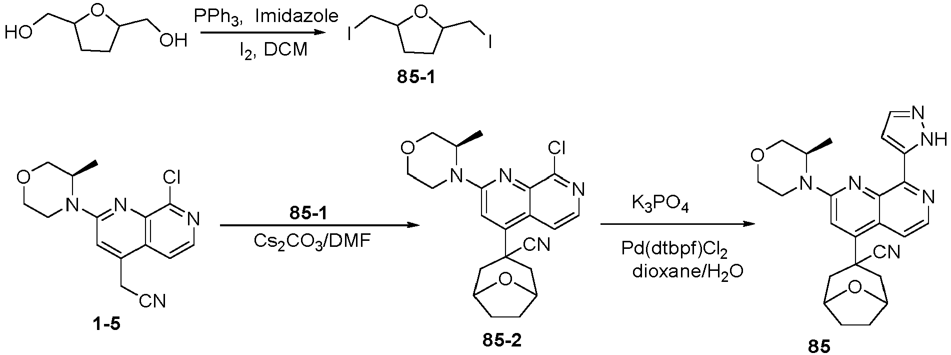

### Step 1: Synthesis of 85-1:

To a 100 mL single-neck flask were added (tetrahydrofuran-2,5-diyl)dimethanol (500 mg, 3.783 mmol), triphenylphosphine (3.97 g, 15.133 mmol), imidazole (1.29 g, 18.915 mmol) and DCM (25 mL). The mixed solution was cooled to 0-5 °C in an ice bath under argon atmosphere, and then iodine (3.84 g, 15.132 mmol) was added in batches. After the addition, the mixed solution was stirred at room temperature overnight. After the completion of the reaction as detected by LC-MS, a saturated sodium thiosulfate solution (25 mL) was added to the mixed solution, the resulting mixture was stirred at room temperature for 30 min, and liquid separation was performed. The aqueous phase was then extracted with DCM (25 mL). The organic phases were combined, washed with saturated sodium chloride solution, dried over anhydrous sodium sulfate and filtered. The filtrate was concentrated, and the residue was purified by Flash to give a product (536 mg, yield: 40.3%).

### Step 2: Synthesis of 85-2:

To a 100 mL single-neck flask were added 1-5 (100 mg, 0.331 mmol) and DMF (10 mL). After the system was purged with argon, Cs₂CO₃ (216 mg, 0.662 mmol) and 85-1 (116 mg, 0.331 mmol) were added. The mixed solution was stirred at room temperature for 20 h. The product was detected by LC-MS, and the mixture was purified by Flash to give a brown oily product (22 mg, yield: 16.7%),

ESI-MS m/z: 399.2 [M+H]⁺.

### Step 3: Synthesis of compound 85:

To a 50 mL single-neck flask were added 85-2 (22 mg, 0.055 mmol), K₃PO₄ (35 mg, 0.165 mmol), pyrazole-3-boronic acid (10 mg, 0.089 mmol), dioxane (5 mL) and water (1 mL). After the system was purged with Ar, Pd(dtbpf)Cl₂ (10 mg, 0.015 mmol) was added. The mixed solution was purged with argon, heated to 80 °C and stirred for 1.5 h. After the completion of the reaction as detected by LC-MS, the mixed solution was filtered and concentrated. The residue was purified by Flash to give a yellowish solid product (6 mg, yield: 25.3%), ESI-MS m/z: 431.2 [M+H]⁺.

**The nuclear magnetic resonance (NMR) of some of the compounds of the present invention are shown in Table 11 below.**

**Table 11**

| **Example** | **NMR** |
|---|---|
| **27** | ¹H NMR (400 MHz, Chloroform-J) δ 8.43 (s, 1H), 7.89 (d, *J* = 5.7 Hz, 1H), 7.76 (s, 1H), 7.38 (s, 1H), 7.24 (s, 1H), 4.44 (d, *J* = 6.8 Hz, 1H), 4.23 - 4.13 (m, 1H), 4.01 (d, *J* = 12.8 Hz, 1H), 3.93 (d, *J* = 11.5 Hz, 1H), 3.85 (dd, *J* = 11.5, 3.1 Hz, 1H), 3.70 (td, *J =* 11.8, 3.0 Hz, 1H), 3.55 (td, *J =* 12.3, 4.6 Hz, 1H), 2.72 (d, *J =* 11.9 Hz, 2H), 2.32 (m, 2H), 2.21 - 2.10 (m, 2H), 2.00 (s, 2H), 1.44 (d, *J* = 6.7 Hz, 3H). |
| **29** | ¹H NMR (400 MHz, cdcl₃) δ 8.49 (d, *J =* 5.7 Hz, 1H), 7.99 (d, *J =* 5.8 Hz, 1H), 7.71 (d, *J =* 1.9 Hz, 1H), 7.32 (d, *J* = 1.8 Hz, 1H), 7.20 (s, 1H), 4.44 (s, 1H), 4.18 (dd, *J* = 12.0, 4.1 Hz, 3H), 4.09 (s, 2H), 4.03 - 3.90 (m, 2H), 3.86 (dd, *J =* 11.4, 3.1 Hz, 1H), 3.71 (td, *J =* 11.7, 3.0 Hz, 1H), 3.56 (td, *J =* 12.3, 3.8 Hz, 1H), 2.42 (d, *J =* 13.2 Hz, 2H), 2.26 (t, *J =* 12.8 Hz, 2H), 1.45 (d, *J =* 6.8 Hz, 3H). |
| **67** | ¹H NMR (400 MHz, cdcl₃) δ 12.76 (s, 1H), 8.49 (d, *J* = 5.7 Hz, 1H), 8.03 (d, *J* = 5.8 Hz, 1H), 7.70 (d, *J* = 1.8 Hz, 1H), 7.31 (d, *J =* 1.8 Hz, 1H), 7.22 (s, 1H), 4.40 (d, *J* = 7.4 Hz, 1H), 4.21 - 4.13 (m, 1H), 3.98 (d, *J =* 12.7 Hz, 1H), 3.92 (d, *J =* 11.5 Hz, 1H), 3.88 - 3.81 (m, 1H), 3.74 - 3.65 (m, 1H), 3.54 (td, *J =* 12.4, 3.9 Hz, 1H), 3.10 (m, 2H), 2.88 (m, 3H), 2.51 (m, 2H), 2.22 (m, 2H), 1.43 (d, *J* = 6.8 Hz, 3H), 1.15 (d, *J* = 6.5 Hz, 6H). |
| **69** | ¹H NMR (400 MHz, cdcl₃) δ 12.73 (s, 1H), 8.49 (d, *J* = 5.7 Hz, 1H), 8.03 (d, *J* = 5.7 Hz, 1H), 7.70 (d, *J =* 1.9 Hz, 1H), 7.31 (s, 1H), 7.21 (s, 1H), 4.39 (s, 1H), 4.20 - 4.14 (m, 1H), 3.98 (d, *J* = 12.5 Hz, 1H), 3.92 (d, *J =* 11.5 Hz, 1H), 3.87 - 3.82 (m, 1H), 3.74 - 3.66 (m, 1H), 3.54 (td, *J =* 12.5, 3.8 Hz, 1H), 3.27 (m, 1H), 2.73 (m, 2H), 2.52 (m, 2H), 1.99 (m, 2H), 1.84 - 1.46 (m, 10H), 1.43 (d, *J* = 6.8 Hz, 3H). |

### Example 86. In- Vitro Anti-Proliferative Activity of Compounds of the Present Invention Against MIA PaCa-2 Cells

MIAPaCa-2 cells were seeded on a 384-well plate at 3000 cells/well. After overnight adherence culture, DMSO or the compounds serially diluted 1:5 from 5 µM were added. The viability was assessed 72 h after dosing by measuring the intracellular ATP content. The inhibition percentage of viable cells by the compounds was calculated by comparing with the DMSO group, and the IC₅₀ value was calculated. The results are shown in Table 12 below.

**Table 12. Anti-proliferative activity of the compounds of the present invention against MIA PaCa-2 cells (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **1** | C | **22** | B | **43** | A | **64** | A |
| **2** | C | **23** | B | **44** | A | **65** | A |
| **3** | C | **24** | B | **45** | A | **66** | A |
| **4** | C | **25** | A | **46** | A | **67** | A |
| **5** | B | **26** | B | **47** | B | **68** | A |
| **6** | B | **27** | B | **48** | A | **69** | A |
| **7** | C | **28** | C | **49** | A | **70** | A |
| **8** | C | **29** | A | **50** | A | **72** | A |
| **9** | B | **30** | B | **51** | A | **73** | A |
| **10** | B | **31** | A | **52** | A | **74** | A |
| **11** | B | **32** | A | **53** | A | **75** | A |
| **12** | B | **33** | A | **54** | A | **76** | A |
| **13** | B | **34** | A | **55** | A | **77** | A |
| **14** | C | **35** | A | **56** | A | **78** | A |
| **15** | C | **36** | A | **57** | A | **80** | A |
| **16** | B | **37** | B | **58** | A | **81** | A |
| **17** | B | **38** | B | **59** | A | **82** | A |
| **18** | B | **39** | B | **60** | A | **83** | A |
| **19** | B | **40** | C | **61** | A | **84** | A |
| **20** | C | **41** | A | **62** | A | **85** | A |
| **21** | B | **42** | A | 63 | A | **BAY1895344** | A |

| | | | | | | | |
|---|---|---|---|---|---|---|---|
| A represents IC₅₀ < 1000 nM B represents 1 µM ≤ IC₅₀ ≤ 5 µM C represents IC₅₀ > 5 µM | | | | | | | |

### Example 87. In- Vitro Anti-Proliferative Activity of Compounds of the Present Invention in Combination with Gemcitabine Against MIA PaCa-2 Cells

MIA PaCa-2 cells were seeded on a 384-well plate at 3000 cells/well, and 20 nM gemcitabine was added. After overnight adherence culture, DMSO or the compounds serially diluted 1:5 from 100 nM were added. The viability was assessed 72 h after dosing by measuring the intracellular ATP content. The inhibition percentage of viable cells by the compounds was calculated by comparing with the DMSO group, and the IC₅₀ value was calculated. The results are shown in Table 13 below.

**Table 13. In-vitro anti-proliferative activity of the compounds of the present invention in combination with gemcitabine against MIA PaCa-2 cells (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **BAY1895344** | 1.71 | **25** | 0.257 | **27** | 1.483 | **29** | 1.499 |
| **33** | 1.079 | **34** | 0.624 | **35** | 0.661 | **43** | 1.236 |

As can be seen from the data in Table 13, the compounds of the present invention, in combination with gemcitabine, have greatly improved *in-vitro* anti-proliferative activity against MIA PaCa-2 cells as compared to the control compound BAY1895344 in combination with gemcitabine.

### Example 88. Liver Microsomal Stability of Some of Compounds of the Present Invention

After 1 µM compound was incubated with 500 g/mL of human or mouse liver microsomes and a NADPH regeneration system at 37 °C for different times, respectively, LC-MS-MS was used to analyze the remaining amount of the compound and calculate T_{1/2}. The results are shown in Table 14 below.

**Table 14. Liver microsomal stability of the compounds**

| **Compound** | **T_{1/2} (min)** | |
|---|---|---|
| | **Human** | **Mouse** |
| **25** | 7.90 | 2.90 |
| **27** | 27.22 | 9.14 |
| **29** | 51.44 | 8.68 |
| **33** | 631.7 | 19.6 |
| **34** | 61.38 | 52.42 |
| **43** | 9.44 | 18.87 |
| **53** | 24.77 | 7.78 |
| **69** | 5.90 | 7.52 |

### Example 89. In Vivo Pharmacokinetic Experiment of Some of Compounds of the Present Invention

CD-1 female mice aged 7 to 10 weeks were intravenously administered and orally administered at a dose of 2 mg/kg and 10 mg/kg, respectively. The mice were fasted for at least 12 h before the administration and given food 4 h after the administration, and they were given *ad libitum* access to water during the experiment.

On the day of the experiment, animals in the intravenous group were administered the corresponding compound by single injection via the tail vein at an administration volume of 10 mL/kg, and animals in the oral group were administered the corresponding compound by single intragastric injection at an administration volume of 10 mL/kg. The animals were weighed before administration, and the administration volume was calculated according to the body weight. The sample collection time was 0.083 h, 0.167 h, 0.5 h, 1 h, 2 h, 4 h, 8 h and 24 h. About 200 µL of whole blood was collected through the orbital venous plexus at each time point and used to prepare plasma for concentration determination by high performance liquid chromatography-tandem mass spectrometry (LC-MS/MS). The plasma concentrations were processed using a non-compartmental model of Winnolin pharmacokinetic software, and the pharmacokinetic parameters were calculated using a linear-log trapezoidal method. The results are shown in Table 15 below.

**Table 15. Results of pharmacokinetic evaluation of compounds in mice**

| **Administration Route** | **Pharmacokinetics Parameter** | **Compound 25** | **Compound 29** | **Compound 33** | **Compound 43** | **Compound 53** |
|---|---|---|---|---|---|---|
| Injection (2 mg/kg) | Vdss (L/Kg) | 1.84 | 0.58 | 1.27 | 1.79 | 1.61 |
| | T_{1/2} (h) | 0.33 | 0.67 | 1.61 | 0.22 | 0.27 |
| | Cl (mL/min/Kg) | 82.02 | 17.4 | 56.65 | 134.99 | 75.14 |
| | AUC₀₋ₗₐₛₜ (h*ng/mL) | 203.21 | 1915.54 | 588.42 | 246.93 | 443.56 |
| Oral administration (10 mg/kg) | Cₘₐₓ (ng/mL) | 455 | 1716.67 | 1007.67 | 616 | 298.67 |
| | Tₘₐₓ (h) | 1 | 0.5 | 0.5 | 0.25 | 0.5 |
| | T_{1/2} (h) | 0.89 | 0.78 | 0.69 | 0.79 | 1.45 |
| | AUC₀₋ₗₐₛₜ (h*ng/mL) | 1103.1 | 3302.52 | 936.66 | 438.60 | 668.95 |
| | F% | 54.3 | 34.46 | 31.84 | 35.52 | 30.16 |

### Example 90. Pharmacokinetic Evaluation of Some of Compounds of the Present Invention in Rats

SPF-grade SD female rats aged 7 to 10 weeks were intravenously administered and orally administered at a dose of 1 mg/kg and 10 mg/kg, respectively. The rats were fasted for at least 12 h before the administration and given food 4 h after the administration, and they were given *ad libitum* access to water during the experiment.

On the day of the experiment, animals in the intravenous group were administered the corresponding compound by single injection via the tail vein at an administration volume of 2 mL/kg, and animals in the oral group were administered the corresponding compound by single intragastric injection at an administration volume of 10 mL/kg. The animals were weighed before administration, and the administration volume was calculated according to the body weight. The sample collection time was 0.083 h (only intravenous group), 0.125 h (only oral group), 0.25 h, 0.5 h, 1 h, 2 h, 4 h, 6 h, 8 h and 24 h. About 200 µL of whole blood (EDTA-K2 anticoagulated) was collected via the jugular vein or other suitable vein at each time point and used to prepare plasma for concentration determination by high performance liquid chromatography-tandem mass spectrometry (LC-MS/MS). All animals were suffocated by CO₂ after the PK samples were collected at the last time point. The plasma concentrations were processed using a non-compartmental model of Winnolin^{™} version 8.2 (Pharsight, mountain View, CA) pharmacokinetic software, and the pharmacokinetic parameters were calculated using a linear-log trapezoidal method.

The results are shown in Table 16 below.

**Table 16. Results of pharmacokinetic evaluation of compounds in rats**

| **Route of Administration** | **Pharmacokinetic parameter** | **Compound 25** | **Compound 29** |
|---|---|---|---|
| Injection (1 mg/kg) | Vdss (L/Kg) | 2.14 | 3.0 |
| | T_{1/2} (h) | 0.65 | 1.71 |
| | Cl (mL/min/Kg) | 61.75 | 24.90 |
| | AUC₀₋ₗₐₛₜ (h*ng/mL) | 269.92 | 669.25 |
| Oral administration (10 mg/kg) | Cₘₐₓ (ng/mL) | 396.33 | 1546.67 |
| | Tₘₐₓ (h) | 0.90 | 1 |
| | T_{1/2} (h) | 0.5 | 2.85 |
| | AUC₀₋ₗₐₛₜ (h*ng/mL) | 1309.43 | 8029.91 |
| | F% | 48.5 | 119.98 |

### Example 91. In Vivo Efficacy Test of Some of Compounds of the Present Invention

Human colon cancer LOVO cells were cultured conventionally in 1640 medium containing 10% fetal bovine serum in an incubator at 37 °C with 5% CO₂. After being subcultured, the cells were collected when they reached the desired amount. 1 × 10⁷ LOVO cells were injected into the left dorsal side of each nude mouse, and the animals were randomly grouped for administration after tumors grew to 100-200 mm³. The groups were as follows: a solvent control group of 8 mice and a compound group of 8 mice. The compounds were administered orally 3 days a week and interrupted for 4 days, for 21 consecutive days. The vehicle was a 0.5% MC suspension containing 1% tween-80. On Tuesday and Thursday each week, tumor volumes and body weight of the mice were measured, and the nude mice were sacrificed on day 21 after the administration. The test results are shown in the table below. The ability of the compounds to inhibit tumor growth was evaluated according to the tumor growth inhibition rate (TGI) = 1 - (tumor volume on day 21 of administration group - tumor volume on day 1 of administration group)/(administration volume on day 21 of control group - tumor volume on day 1 of control group). The toxicity of the compounds was evaluated according to the body weight of the mice. The groups were as follows:
1) a solvent control group; 2) a compound 25 group; 3) a compound 27 group; 4) a compound 29 group; 5) a compound 33 group; 6) a compound 34 group; 7) a compound 43 group; 8) a control compound (BAY1895344) group.

The results are shown in Table 17 below.

**Table 17. In vivo efficacy of compounds in LoVo model**

| **Group** | **Compound** | **Dose** | **Administration period** | **Route of Administration** | **TGI(%)** |
|---|---|---|---|---|---|
| 1 | Vehicle | -- | Days 1-3, days 8-10, days 15-17, and day 21 | PO | -- |
| 2 | **25** | 20mpk BID | | PO | 84.0% |
| 3 | **27** | 20mpk BID | | PO | 62.7% |
| 4 | **29** | 20mpk BID | | PO | 83.2% |
| 5 | **33** | 20mpk BID | | PO | 79.1% |
| 6 | **34** | 20mpk BID | | PO | 81.4% |
| 7 | **43** | 20mpk BID | | PO | 75.3% |
| 8 | **BAY1895344** | 20mpk BID | | PO | 71.2% |

### Example 92. In Vivo Efficacy Test of Some of Compounds of the Present Invention

Human colon cancer HT29 cells were cultured conventionally in 1640 medium containing 10% fetal bovine serum in an incubator at 37 °C with 5% CO₂. After being subcultured, the cells were collected when they reached the desired amount. 1 × 10⁷ HT29 cells were injected into the left dorsal side of each nude mouse, and the animals were randomly grouped for administration after tumors grew to 100-200 mm³. The groups were as follows: a solvent control group of 6 mice, a single drug group of 15 mg/kg gemcitabine (GMC), and a combination group of 15 mg/kg gemcitabine and a compound. Gemcitabine was administered intraperitoneally with PBS as a vehicle once a week. The compounds were administered 3 days a week and interrupted for 4 days (3 on/4 off), for 21 consecutive days, and the vehicle was a 0.5% MC suspension containing 1% tween-80. On Tuesday and Thursday each week, tumor volumes and body weight of the mice were measured, and the nude mice were sacrificed on day 21 after the administration. The test results are shown in the table below. The ability of the compounds to inhibit tumor growth was evaluated according to the tumor growth inhibition rate (TGI) = 1 - (tumor volume on day 21 of administration group - tumor volume on day 1 of administration group)/(administration volume on day 21 of control group - tumor volume on day 1 of control group). The toxicity of the compounds was evaluated according to the body weight of the mice.

The groups were as follows:
1) a solvent control group; 2) a solvent + GMC group; 3) a compound 25 group; 4) a compound 25 + GMC group; 5) a compound 29 group; 6) a compound 29 + GMC group; 7) a compound 33 group; 8) a compound 33 + GMC group; 9) a compound 34 group; 10) a compound 34 + GMC group; 11) a compound 43 group; 12) a compound 43 + GMC group; 13) a control compound (BAY1895344) group; 14) a control compound (BAY1895344) + GMC group.

The results are shown in Table 18 below.

**Table 18. In vivo efficacy of some of compounds of the present invention in HT-29 model**

| **Group** | **Compound** | **Dose** | **Administration period** | **Route of Administration** | **TGI(%)** |
|---|---|---|---|---|---|
| 1 | Vehicle | -- | 3on/4off | PO | -- |
| 2 | GMC | 15mpk QW | Once a week | IP | 50.7% |
| 3 | 25 | 20mpk BID | 3on/4off | PO | 53.1% |
| 4 | 25+ | 20mpk BID | 3 on/4 off | PO | 86.1% |
| | GMC | 15mpk QW | Once a week | IP | |
| 5 | 29 | 20mpk BID | 3on/4off | PO | 49.9% |
| 6 | 29+ | 20mpk BID | 3 on/4 off | PO | 85.3% |
| | GMC | 15mpk QW | Once a week | IP | |
| 7 | 33 | 20mpk BID | 3on/4off | PO | 57.4% |
| 8 | 33+ | 20mpk BID | 3 on/4 off | PO | 82.3% |
| | GMC | 15mpk QW | Once a week | IP | |
| 9 | 34 | 20mpk BID | 3on/4off | PO | 58.3% |
| 10 | 34+ | 20mpk BID | 3 on/4 off | PO | 78.1% |
| | GMC | 15mpk QW | Once a week | IP | |
| 11 | 43 | 20mpk BID | 3on/4off | PO | 51.2% |
| 12 | 43+ | 20mpk BID | 3 on/4 off | PO | 84.4% |
| | GMC | 15mpk QW | Once a week | IP | |
| 13 | BAY1895344 | 20mpk BID | 3on/4off | PO | 53.1% |
| 14 | BAY1895344+ | 20mpk BID | 3 on/4 off | PO | 74.3% |
| | GMC | 15mpk QW | Once a week | IP | |

Although specific embodiments of the present invention have been described above, it will be appreciated by those skilled in the art that these embodiments are merely illustrative and that many changes or modifications can be made to these embodiments without departing from the principles and spirit of the present invention. The protection scope of the present invention is therefore defined by the appended claims.

## Claims

1. A compound of general formula (1) or an isomer, a crystalline form, a pharmaceutically acceptable salt, a hydrate or a solvate thereof: 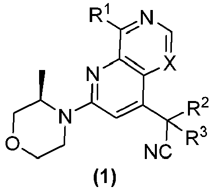 wherein in general formula (1):
X is CH or N;
R¹ is 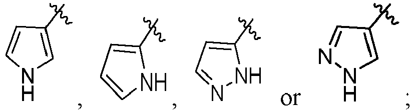
R² and R³ are each independently -H, -D, halogen, (C1-C6) alkyl, (C2-C6) alkenyl, (C2-C6) alkynyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl, wherein the (C1-C6) alkyl, (C2-C6) alkenyl, (C2-C6) alkynyl, (C1-C6) alkoxy, (C3-C10) cycloalkyl, (C3-C10) cycloalkenyl, (3- to 10-membered) heterocycloalkyl, (3-to 10-membered) heterocycloalkenyl, (C6-C10) aryl or (5-to 10-membered) heteroaryl can each independently be optionally substituted with one or more of the following groups: -H, -D, halogen, -OH, -R⁶, -NR⁴R⁵, -C(O)OR⁴, -C(O)NR⁴R⁵, -S(O)ₚR⁴, -S(O)₂NR⁴R⁵, -P(O)(OR⁴)₂, - P(O)(R⁴)₂, (C1-C6) alkyl, (C2-C6) alkenyl, (C2-C6) alkynyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
or R² and R³, together with the carbon atom to which they are attached, form a (C3-C15) cycloalkyl or (3- to 15-membered) heterocycloalkyl, wherein the (C3-C15) cycloalkyl or (3- to 15-membered) heterocycloalkyl can each independently be optionally substituted with one or more R⁶;
R⁴ and R⁵ are each independently -H, -D, (C1-C3) alkyl, (C2-C4) alkenyl, (C2-C4) alkynyl, (C1-C3) alkoxy, (C1-C3) haloalkyl, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
or R⁴ and R⁵, with the attached N atom, form a (3- to 10-membered) heterocycloalkyl, wherein the (3- to 10-membered) heterocycloalkyl can be optionally substituted with one or more of the following groups: -H, -D, halogen, -OH, -NR⁷R⁸, -C(O)OR⁷, -C(O)NR⁷R⁸, -S(O)ₚR⁷, - S(O)₂NR⁷R⁸, -P(O)(OR⁷)₂, -P(O)(R⁷)₂, (C1-C6) alkyl, (C2-C6) alkenyl, (C2-C6) alkynyl, (Cl-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
each R⁶ is independently -H, -D, halogen, -OH, -NR⁷R⁸, -C(O)R⁷, -C(O)OR⁷, -C(O)NR⁷R⁸, - (CH₂)ₙ-S(O)ₚR⁷, -(CH₂)ₙ-S(O)₂NR⁷R⁸, -P(O)(OR⁷)₂, -P(O)(R⁷)₂, (C1-C6) alkyl, (C2-C6) alkenyl, (C2-C6) alkynyl, (C1-C6) haloalkyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl, wherein the (C1-C6) alkyl, (C2-C6) alkenyl, (C2-C6) alkynyl, (C1-C6) haloalkyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl can each independently be optionally substituted with one or more of the following groups: -H, -D, halogen, -OH, -NR⁷R⁸, -C(O)OR⁷, -C(O)NR⁷R⁸, -(CH₂)ₙ-S(O)ₚR⁷, -(CH₂)ₙ-S(O)₂NR⁷R⁸, -P(O)(OR⁷)₂, -P(O)(R⁷)₂, (C1-C6) alkyl, (C2-C6) alkenyl, (C2-C6) alkynyl, (C1-C6) haloalkyl, (C1-C6) alkoxy, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
R⁷ and R⁸ are each independently -H, -D, (C1-C3) alkyl, (C2-C4) alkenyl, (C2-C4) alkynyl, (C1-C3) alkoxy, (C1-C3) haloalkyl, (C3-C10) cycloalkyl, (C3-C10) cycloalkenyl, (3- to 10-membered) heterocycloalkyl, (3- to 10-membered) heterocycloalkenyl, (C6-C10) aryl or (5- to 10-membered) heteroaryl;
p is 0, 1 or 2;
n is 0, 1, 2 or 3.

2. The compound or the isomer, the crystalline form, the pharmaceutically acceptable salt, the hydrate or the solvate thereof according to claim 1, wherein in general formula (1), X is CH.

3. The compound or the isomer, the crystalline form, the pharmaceutically acceptable salt, the hydrate or the solvate thereof according to claim 1 or 2, wherein in general formula (1), R¹ is

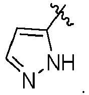

4. The compound or the isomer, the crystalline form, the pharmaceutically acceptable salt, the hydrate or the solvate thereof according to any one of claims 1-3, wherein in general formula (1), R² and R³ are each independently -H, -D, -F, -Cl, -Br, -I, (C1-C3) alkyl, (C2-C4) alkenyl, (C2-C4) alkynyl, (C1-C3) alkoxy, (C3-C6) cycloalkyl, (C3-C6) cycloalkenyl, (3- to 8-membered) heterocycloalkyl, (3- to 8-membered) heterocycloalkenyl, (C6-C10) aryl or (5- to 10-membered) heteroaryl, wherein the (C1-C3) alkyl, (C2-C4) alkenyl, (C2-C4) alkynyl, (C1-C3) alkoxy, (C3-C6) cycloalkyl, (C3-C6) cycloalkenyl, (3- to 8-membered) heterocycloalkyl, (3-to 8-membered) heterocycloalkenyl, (C6-C10) aryl or (5- to 10-membered) heteroaryl can each independently be optionally substituted with one or more of the following groups: -H, -D, -F, - Cl, -Bᵣ, -I, -OH, -R⁶, -NR⁴R⁵, -C(O)OR⁴, -C(O)NR⁴R⁵, -S(O)ₚR⁴, -S(O)₂NR⁴R⁵, -P(O)(OR⁴)₂, - P(O)(R⁴)₂, (C1-C3) alkyl, (C2-C4) alkenyl, (C2-C4) alkynyl, (C1-C3) alkoxy, (C3-C6) cycloalkyl, (C3-C6) cycloalkenyl, (3- to 8-membered) heterocycloalkyl, (3- to 8-membered) heterocycloalkenyl, (C6-C10) aryl or (5- to 10-membered) heteroaryl; or R² and R³, together with the carbon atom to which they are attached, form a (C3-C15) cycloalkyl or (3- to 15-membered) heterocycloalkyl, wherein the (C3-C15) cycloalkyl or (3- to 15-membered) heterocycloalkyl can each independently be optionally substituted with one or more R⁶.

5. The compound or the isomer, the crystalline form, the pharmaceutically acceptable salt, the hydrate or the solvate thereof according to any one of claims 1-4, wherein in general formula (1), R² and R³ are each independently -H, -F, -Cl, -CH₃,

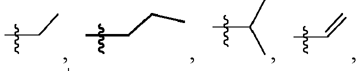

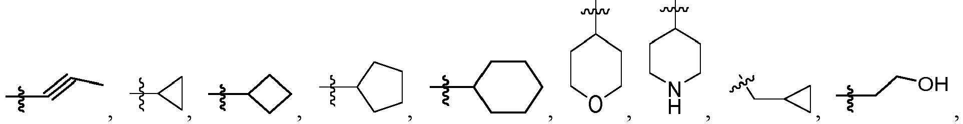

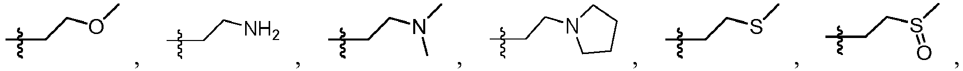

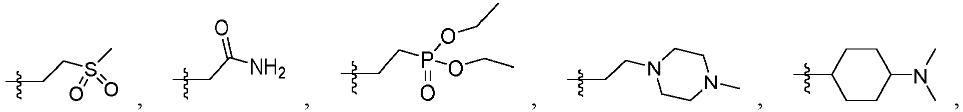

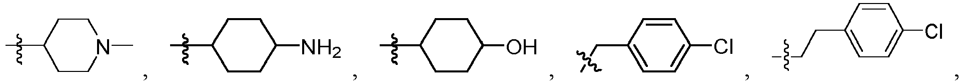

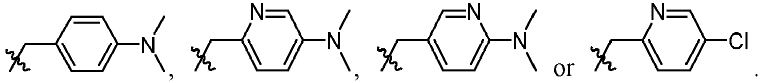

6. The compound or the isomer, the crystalline form, the pharmaceutically acceptable salt, the hydrate or the solvate thereof according to any one of claims 1-4, wherein in general formula (1), the (C3-C15) cycloalkyl or (3- to 15-membered) heterocycloalkyl formed by R² and R³ together with the carbon atom to which they are attached is: 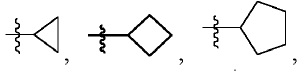 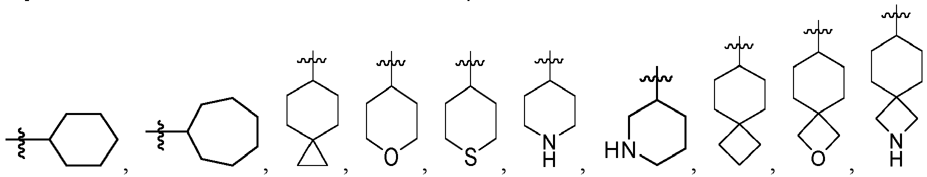 or 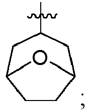 the (C3-C15) cycloalkyl or (3- to 15-membered) heterocycloalkyl can be optionally substituted with one or more of the following groups: -H, -F, -CH₃, -CH₂CH₃, -OH, 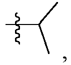 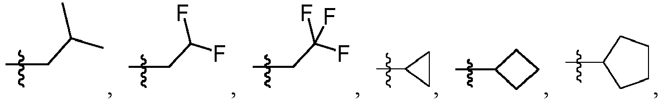 -NH₂, -NH(CH₃), -N(CH₃)₂, - N(CH₂CH₃)₂, -OCH₃, -OCH₂CH₃,

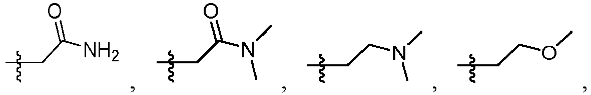

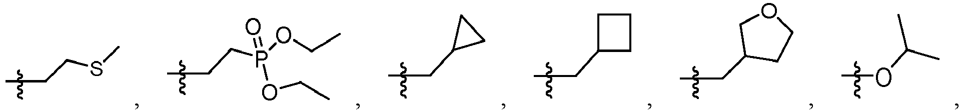

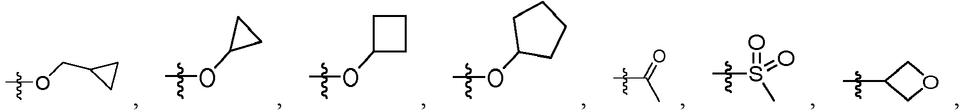

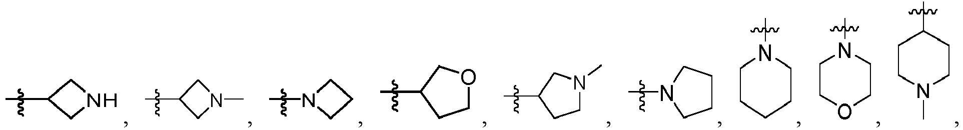

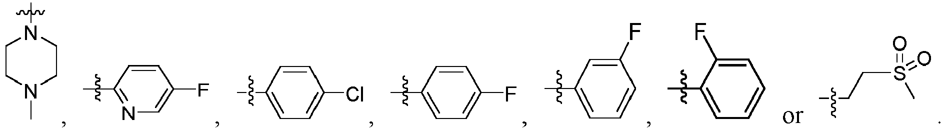

7. The compound or the isomer, the crystalline form, the pharmaceutically acceptable salt, the hydrate or the solvate thereof according to any one of claims 1-4, wherein in general formula (1), R⁴ and R⁵ are each independently -H, -D, -CH₃,

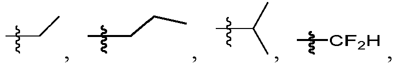

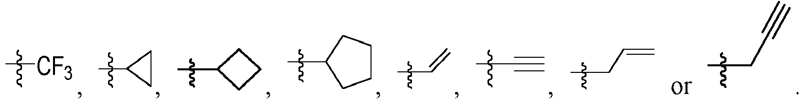

8. The compound or the isomer, the crystalline form, the pharmaceutically acceptable salt, the hydrate or the solvate thereof according to claim 7, wherein in general formula (1), R⁴ and R⁵ are each independently -H or -CH₃.

9. The compound or the isomer, the crystalline form, the pharmaceutically acceptable salt, the hydrate or the solvate thereof according to any one of claims 1-4, wherein in general formula (1), R⁶ is -H, -F, -CH₃, -CH₂CH₃, -OH, 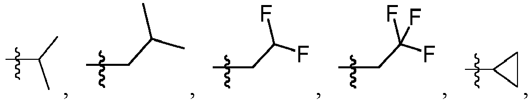 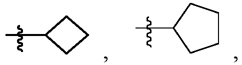 -NH₂, -NH(CH₃), -N(CH₃)₂, -N(CH₂CH₃)₂, -OCH₃, -OCH₂CH₃,

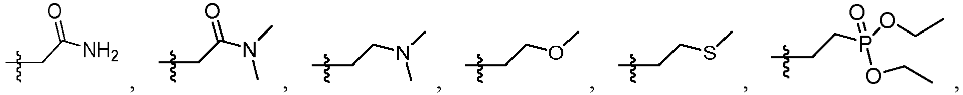

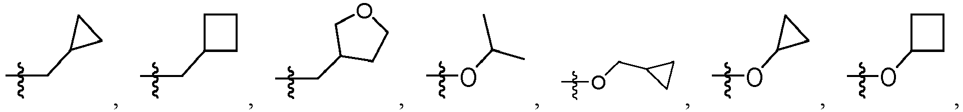

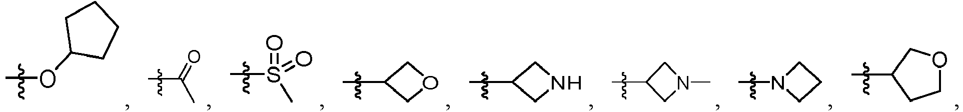

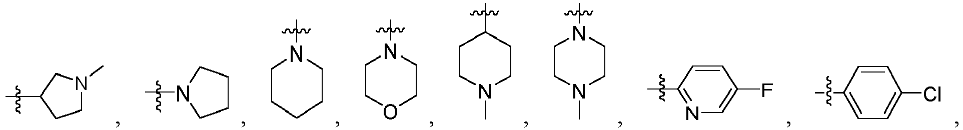

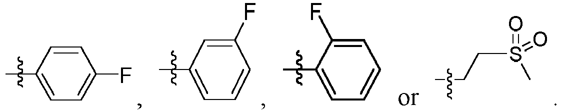

10. The compound or the isomer, the crystalline form, the pharmaceutically acceptable salt, the hydrate or the solvate thereof according to any one of claims 1-4, wherein in general formula (1), R⁷ and R⁸ are each independently -H or -CH₃.

11. The compound or the isomer, the crystalline form, the pharmaceutically acceptable salt, the hydrate or the solvate thereof according to any one of claims 1-10, wherein the compound has one of the following structures:

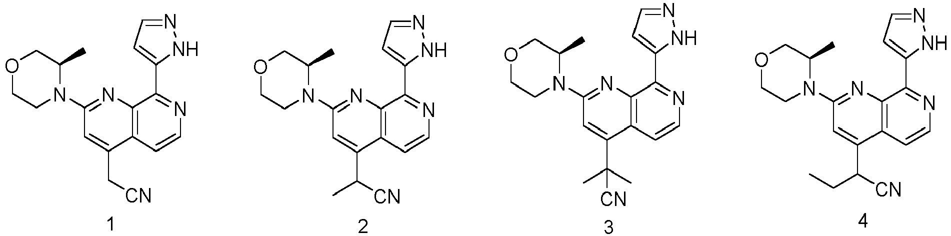

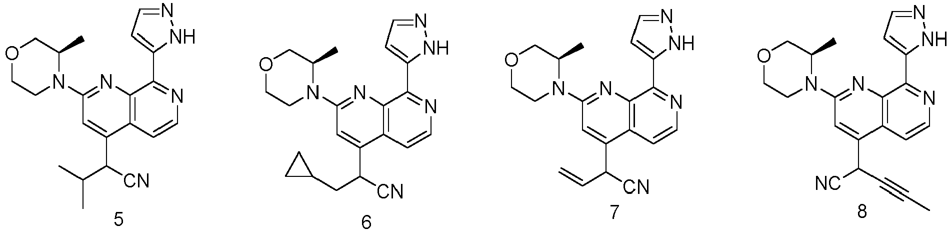

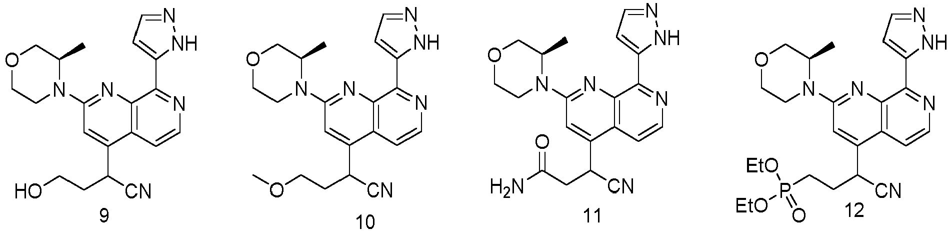

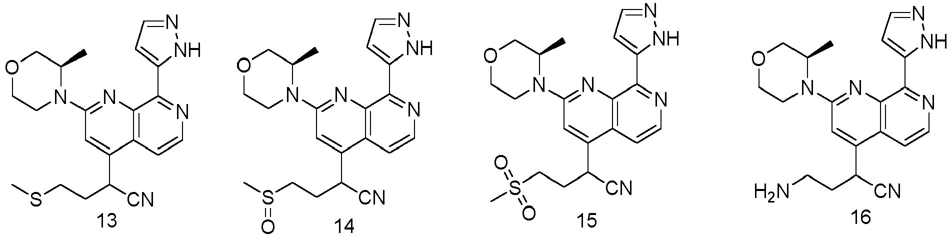

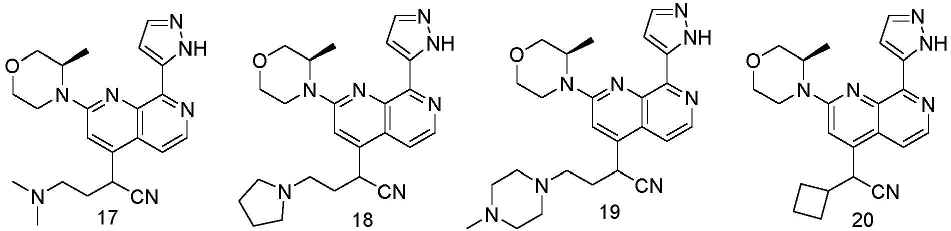

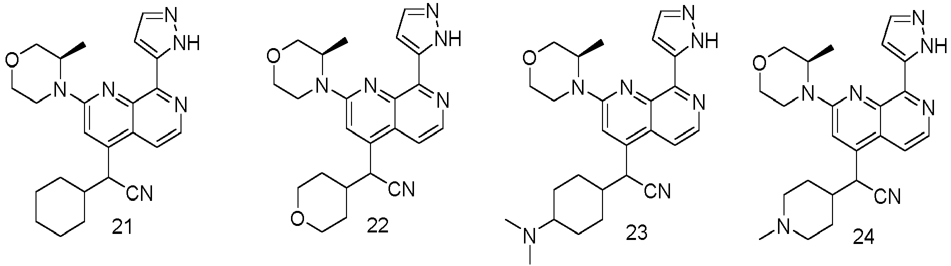

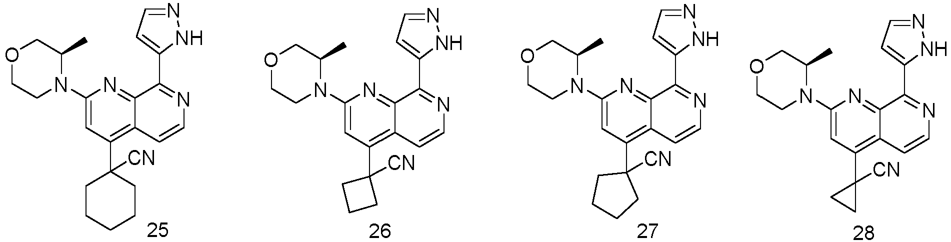

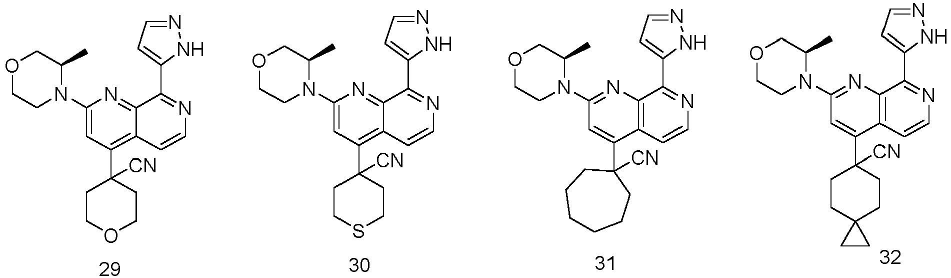

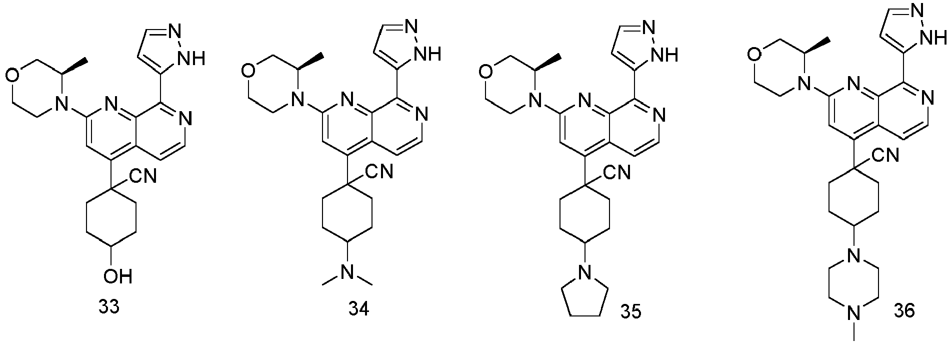

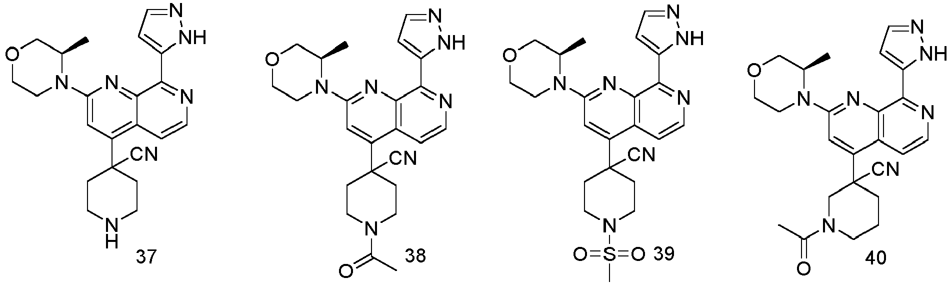

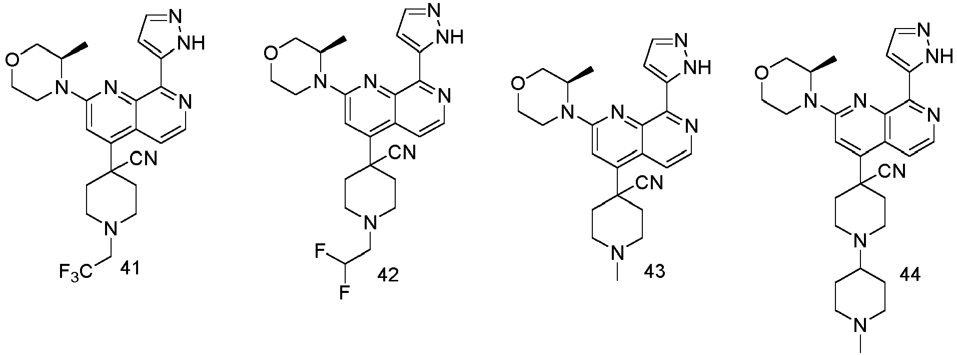

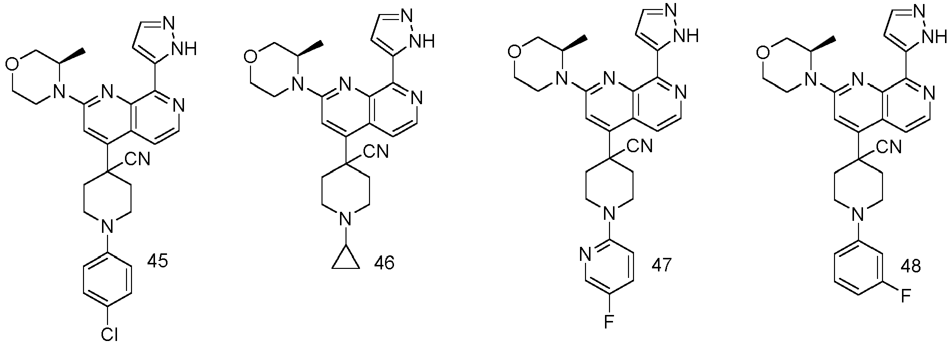

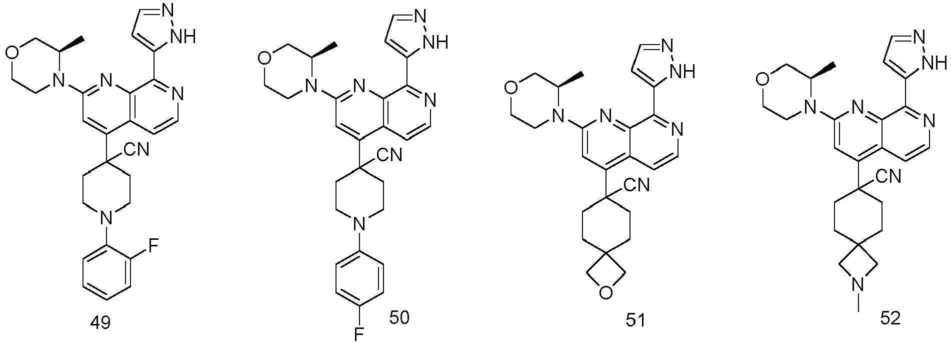

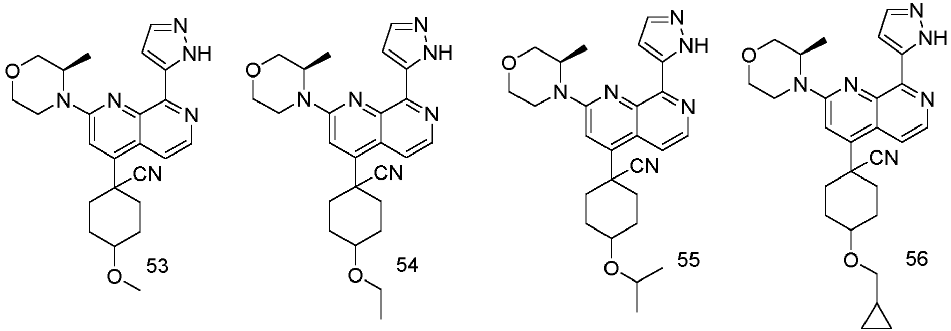

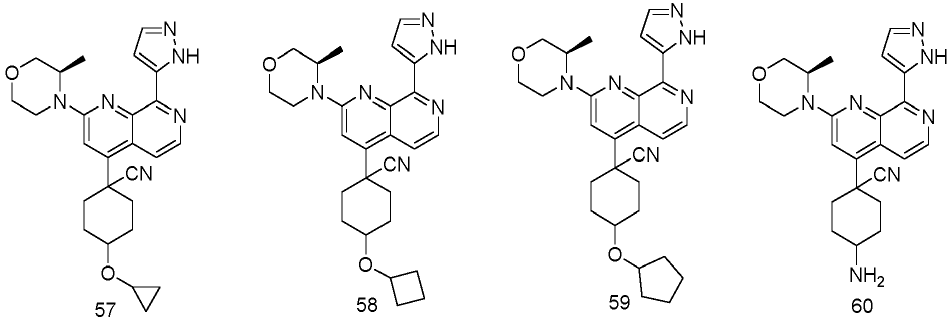

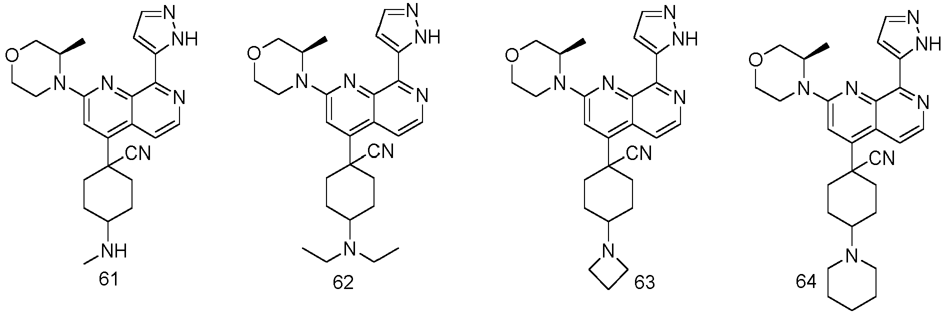

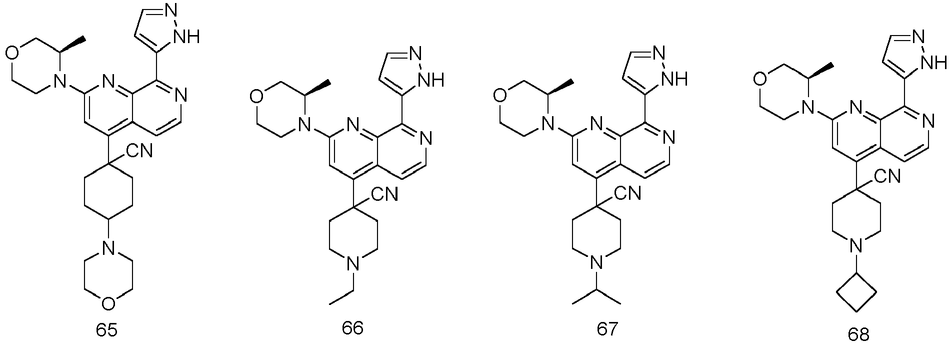

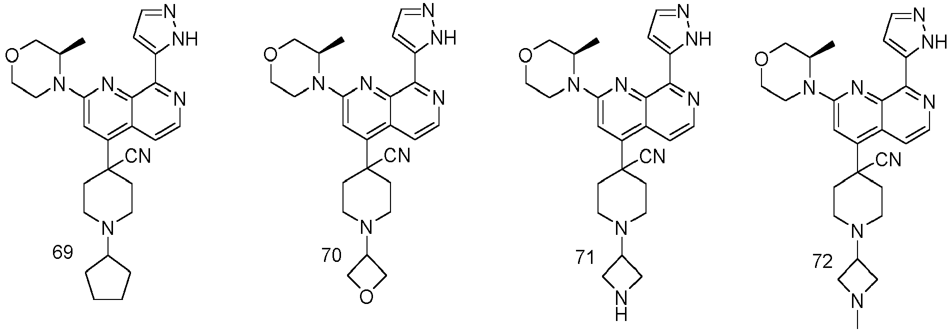

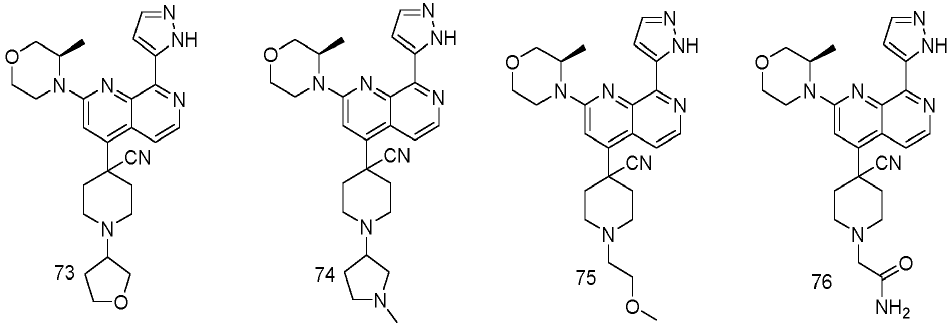

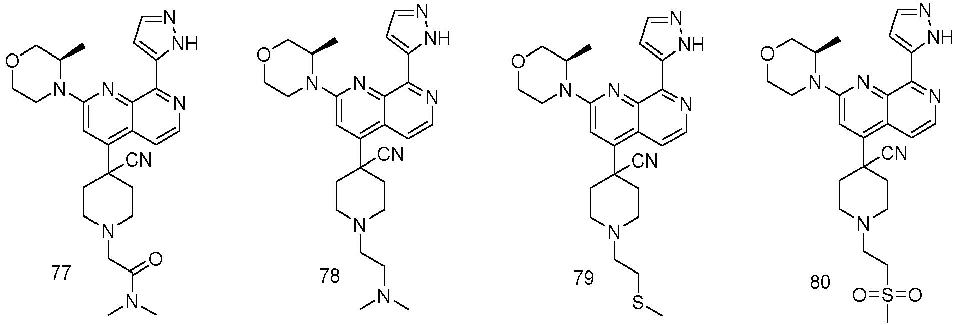

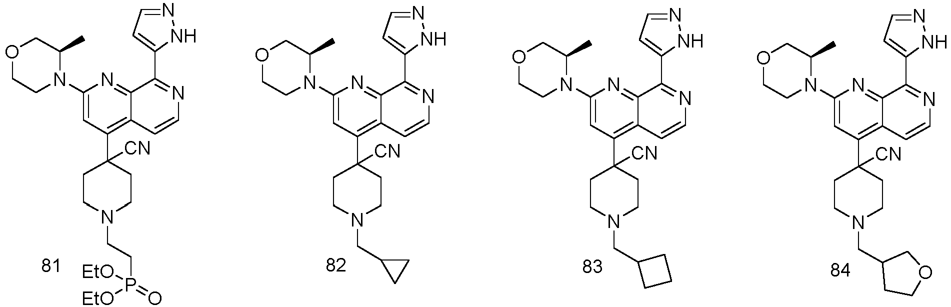

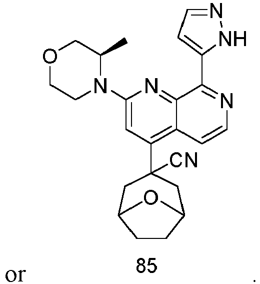

12. A pharmaceutical composition, comprising a pharmaceutically acceptable excipient or carrier, and the compound or the isomer, the crystalline form, the pharmaceutically acceptable salt, the hydrate or the solvate thereof according to any one of claims 1-11 as an active ingredient.

13. Use of the compound or the isomer, the crystalline form, the pharmaceutically acceptable salt, the hydrate or the solvate thereof according to any one of claims 1-11, or the pharmaceutical composition according to claim 12 in the preparation of a medicament for treating a disease related to ATR protein kinase.

14. The use according to claim 13, wherein the disease is cancer, and the cancer is a hematologic cancer or a solid tumor.
